(19) Europäisches Patentamt / European Patent Office / Office européen des brevets

(11) **EP 3 733 143 B1**

(12) **EUROPEAN PATENT SPECIFICATION**

(45) Date of publication and mention
of the grant of the patent:
**15.04.2026 Bulletin 2026/16**

(21) Application number: **18894352.6**

(22) Date of filing: **18.01.2018**

(51) International Patent Classification (IPC):
**A61F 13/534** *(2006.01)* **A61F 13/539** *(2006.01)*
**A61F 13/532** *(2006.01)* **A61F 13/537** *(2006.01)*

(52) Cooperative Patent Classification (CPC):
**A61F 13/5323; A61F 13/534; A61F 13/537;
A61F 13/53752**

(86) International application number:
**PCT/JP2018/001388**

(87) International publication number:
**WO 2019/130601 (04.07.2019 Gazette 2019/27)**

(54) **ABSORBENT ARTICLE**

SAUGFÄHIGER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.12.2017 CN 201711431157**

(43) Date of publication of application:
**04.11.2020 Bulletin 2020/45**

(73) Proprietor: **Daio Paper Corporation
Ehime 799-0492 (JP)**

(72) Inventors:
• **MANABE, Sadanao
Tokyo 102-0071 (JP)**
• **OCHI, Ryoichi
Shikokuchuo-shi, Ehime 799-0431 (JP)**
• **MATSUI, Tomotsugu
Nantong, Jiangsu 226010 (CN)**
• **LIU, Hong Yan
Nantong, Jiangsu 226010 (CN)**
• **WANG, Xiao Xia
Nantong, Jiangsu 226010 (CN)**
• **LIU, Xiang Qian
Nantong, Jiangsu 226010 (CN)**

(74) Representative: **Meissner Bolte Partnerschaft
mbB
Patentanwälte Rechtsanwälte
Postfach 86 06 24
81633 München (DE)**

(56) References cited:
**WO-A1-2014/170859    WO-A2-2015/002934
JP-A- 2017 176 507    JP-A- 2017 176 507
JP-A- H06 190 002    JP-A- H06 190 003
JP-A- H06 315 501    JP-A- H06 315 501
US-A1- 2013 079 741**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 3 733 143 B1

## Description

### Technical Field

The present invention relates to an absorbent article such as a disposable diaper and a sanitary napkin.

### Background Art

The absorbent article includes an absorber and a liquid-pervious top sheet covering the front surface side of the absorber. Excretion liquid such as urine and menstrual blood passes through the top sheet and is absorbed and held by the absorber. An absorber obtained by mixing super absorbent polymer (SAP) particles with hydrophilic short fibers such as fluff pulp and accumulating them in a cotton form has been widely used. However, in response to the request for further thinning, weight reduction, and cost reduction while ensuring a sufficient absorbable amount, various types of absorbers (hereinafter also referred to as cell absorbers) are proposed. Such an absorber includes a large number of cells (small chambers), which are surrounded by bonded portions of the liquid pervious upper sheet and the lower sheet and in each of which the upper sheet and the lower sheet are not bonded, and particulate materials including super absorbent polymer particles contained in the cells (for example, refer to Patent Literatures 1 to 6 below).

WO 2014/170859 A1 discloses an absorbent sanitary article comprising a permeable inner layer, an impermeable outer layer, an absorbent pad interposed between the inner layer and the outer layer and comprising a first layer of non-woven fabric, a second layer of non-woven fabric partly joined to the first layer of non-woven fabric by a plurality of seals, an absorbent core interposed between the first and second layers of non-woven fabric, the absorbent core comprising a spread of absorbent polymer material. The absorbent core further comprises a third layer of non-woven fabric interposed between the first and second layers of non-woven fabric.

US 2013/079741 A1 discloses an absorbent article having a liquid-permeable top sheet, a liquid-impermeable back sheet and an absorbent body positioned between both of these sheets, wherein the absorbent body is composed of a superabsorbent polymer-containing layer and a non-woven fabric layer, the non-woven fabric layer has regions where inter-fiber void ratio is relatively large and regions where inter-fiber void ratio is relatively small, the ends of the fibers that compose the non-woven fabric layer are more numerous in those regions where the inter-fiber void ratio is relatively large than in those regions where the inter-fiber void ratio is relatively small, and the nonwoven fabric layer has expansibility.

JP06-315501 A also discloses water absorptive sheet to be used for physiological articles, paper diapers, etc., formed by interposing water absorptive resin particles between support sheets having water permeability.

In such a cell absorber, when the volume of the super absorbent polymer particles in the state of saturated absorption in each cell is sufficiently larger than the volume of the cell, the super absorbent polymer particles fill an inside of the cell upon the absorption, and there is concern that the absorption amount and absorption rate may be lowered due to inhibition by the swelling and so-called gel blocking, the wearing feeling may be deteriorated since the cell becomes hard due to the swelling pressure of the super absorbent polymer particles, and the super absorbent polymer particles may leak from gaps among fibers in a case where the upper sheet and the lower sheet is formed of nonwoven fabrics.

For this reason, in the cell absorber, at least one of the upper sheet and the lower sheet in the cell is formed into a concave depressed outside the cell, and a large cell volume is ensured (for example, refer to Patent Literatures 5 and 6 below). In addition, in order to be able to cope with swelling of the super absorbent polymer particles to individual cell volumes or more, a structure is proposed in which the bonded portions are peeled off during absorption, and the volume of each cell is enlarged by coalescence of adjacent cells (for example, refer to Patent Literatures 1 to 6 below).

However, when compared to an absorber obtained by mixing the super absorbent polymer particles with hydrophilic short fibers such as fluff pulp and accumulating them in a cotton form, further improvement is desired in the absorption rate of the cell absorber.

### Citation List

#### Patent Literature

Patent Literature 1: JP 09-504207 A
Patent Literature 2: JP 2014-500736 A
Patent Literature 3: JP 2011-189067 A
Patent Literature 4: JP 10-137291 A
Patent Literature 5: JP 2017-176507 A
Patent Literature 6: JP 2010-522595 A

Summary of Invention

Technical Problem

Therefore, a main object of the invention is to improve the absorption rate in the absorbent article including the cell absorber.

Solution to Problem

The invention solving the above-mentioned problem is defined is set forth in the independent claim. Embodiments result from the dependent claims and the below description.

The absorbent article that solves the above problems is as follows.

<First Aspect>

An absorbent article including

an absorber having an upper sheet, a lower sheet disposed on a back surface side of the upper sheet, a plurality of cells each surrounded by bonded portions of the upper sheet and the lower sheet, the upper sheet and the lower sheet not bonded in each cell, a particulate material including super absorbent polymer particles contained in each cell, and a concave depressed to outsides of each cell in a spread state being formed by embossing the upper sheet at each cell,

in which a middle sheet made of a nonwoven fabric is interposed between the upper sheet and the lower sheet, and the middle sheet is compressed in a thickness direction at the bonded portions and is expanded to inside of the concave within the cell,

a surface of the middle sheet facing the concave formed in the upper sheet is in contact with an inner surface of the concave formed in the upper sheet, or is spaced apart from the inner surface of the concave for a separation distance of 30 % or less of a depth of the concave formed in the upper sheet, and the super absorbent polymer particles in each cell are present most on an upper surface of the middle sheet and decrease downward therefrom.

Further, in addition to the concaves provided in the upper sheet, concaves may also be formed in the lower sheet, wherein the super absorbent polymer particles in each cell are in particular present most on the upper surface of the lower sheet and decrease upward therefrom.

(Function and Effect)

According to findings of the present inventor, in the cell absorber, even when the upper sheet in the cells is formed into concaves depressed to the outsides of the cells to ensure large volumes of the cells, if the concaves are crushed by the pressure applied in the package state of the product or the pressure applied during wearing, there is concern that shapes of the concaves may be difficult to recover and swelling of the super absorbent polymer particles may be hindered. The inhibition of swelling occurs at an initial stage of absorption, and thus has a large effect on the absorption rate.

On the other hand, in the present aspect, since the middle sheet made of the nonwoven fabric is compressed in the thickness direction at the bonded portions and expands to the insides of the concaves at portions located inside the cells, the concaves are rarely crushed by the pressure applied in the package state of the product or the pressure applied during wearing. Even when the concaves are crushed, elasticity of the middle sheet promotes restoration of the shapes of the concaves to at least a portion where the middle sheet enters or a volume close to the portion. Further, during absorption of the excretion liquid, the super absorbent polymer expands the fiber gap and can swell while entering the gap, easily compressing the middle sheet, or entering the gap and compressing the middle sheet, and thus the presence of the middle sheet is unlikely to hinder swelling of the super absorbent polymer particles. Therefore, by the synergistic action thereof, the absorption rate of the absorbent article including the cell absorber of the present aspect (particularly, an initial stage of absorption) is improved.

The super absorbent polymer particles in the cells are most present on an upper surface of the middle sheet and decrease downward therefrom.

(Function and Effect)

The distribution of the super absorbent polymer particles in the cells is not particularly limited. However, with the distribution of the present aspect, when the user touches the outer surface of the absorbent article with hand, scratchy feeling (uncomfortable feeling) of the super absorbent polymer particles is rarely transmitted to the hand due to intervention of the middle sheet. In particular, when the middle sheet is a bulky nonwoven fabric having a high porosity, since the super absorbent polymer particles can enter the fiber gap of the middle sheet before absorption of the excretion liquid and during absorption of the excretion liquid, the absorption rate is further improved. That **is,** in an initial stage of the absorption, the absorption proceeds on the upper surface of the middle sheet where a large number of the super absorbent polymer particles are distributed, and a rate thereof is limited. Therefore, in the initial stage of the absorption, a large amount of excretion liquid penetrates into the middle sheet having a small number of the super absorbent polymer particles, and absorbed by the super absorbent polymer particles in the middle sheet, temporarily stored until absorbed by the super absorbent polymer particles, or diffuse to the surrounding cells. The excretion liquid diffused to the surroundings is absorbed by the super absorbent polymer particles in the middle sheet existing there or sucked up by the super absorbent polymer particles abundant thereabove. Then, in a process in which each of the super absorbent polymer particles absorbs the excretion liquid, the super absorbent polymer expands the fiber gap and swells while penetrating into the gap or compressing the middle sheet. Due to such an absorption mechanism, the excretion liquid rapidly spreads over a wide area of the cell absorber and is in a state of being received inside the cell absorber. Thus, not only the absorption rate is improved, but also a reflowing prevention property is excellent.

<Second Aspect>

The absorbent article according to the first aspect,

in which the super absorbent polymer particles are not attached to any of the upper sheet, the middle sheet, and the lower sheet, and
a surface of the middle sheet facing the concaves is in contact with inner surfaces of the concaves.

(Function and Effect)

When the cells have hollow portions as described in Patent Literature 6, the super absorbent polymer particles move in the cells during use. Thus, there is concern that noise may be generated or absorption may be inhibited due to uneven distribution of the super absorbent polymer particles in the cells. To solve this problem, when the super absorbent polymer particles are fixed using a watersoluble adhesive as described in Patent Literature 6, swelling of the super absorbent polymer particles in the initial stage of absorption is hindered, and thus, there is concern that the absorption rate may be reduced.
On the other hand, as in the present aspect, when the super absorbent polymer particles do not adhere to each other, and the surface of the middle sheet facing the concaves is brought into contact with the inner surfaces of the concaves, in other words, almost all of the cells including the concaves are filled with the fibers of the middle sheet, the super absorbent polymer particles are trapped by the fibers of the middle sheet, or pressed against the upper sheet or the lower sheet, or trapped and pressed, and thus free movement is less likely to occur. Therefore, while preventing inhibition of swelling of the super absorbent polymer particles, it is possible to prevent generation of noise due to movement of the super absorbent polymer particles and inhibition of absorption due to uneven distribution of the super absorbent polymer particles in the cells.

<Third Aspect>

The absorbent article according to the first or second aspect, in which the bonded portions are provided in a dotted line pattern along a direction of surrounding the cells.

(Function and Effect)

In the present aspect, a fiber group of the middle sheet passes between adjacent bonded portions and extends between a large number of cells. Therefore, since a liquid diffusion passage is formed between the adjacent bonded portions, the liquid diffusibility between cells is improved, so that the absorption rate is improved.

<Fourth Aspect>

The absorbent article according to any one of the first to third aspects,

in which the bonded portions include weak bonded portions allowed to be peeled off by swelling forces of super absorbent polymer particles in adjacent cells, and

a peel strength of one of a facing surface between the upper sheet and the middle sheet and a facing surface between the middle sheet and the lower sheet is lower than a peel strength of the other one in the weak bonded portions.

(Function and Effect)

To cope with swelling of the super absorbent polymer particles to individual cell volumes or more, it is desirable that the bonded portions are peeled off during absorption, and the volume of each cell is enlarged by coalescence of adjacent cells. However, in a case where the middle sheet is provided between the upper sheet and the lower sheet, when there are weak bonded portions where the upper sheet and the middle sheet peel off first, or weak bonded portions where the middle sheet and the lower sheet peel off first in the bonded portions, the shape of swelling of the cell absorber becomes irregular, and there is concern that swelling may be hindered or the wearing feeling may deteriorate. On the other hand, when peeling of one of the facing surface between the upper sheet and the middle sheet and the facing surface between the middle sheet and the lower sheet preferentially occurs in the weak bonded portions as in the present aspect, the cell absorber swells uniformly and smoothly, which is preferable.

<Fifth Aspect>

The absorbent article according to any one of the first to fourth aspects, in which the middle sheet is formed of a nonwoven fabric having a fineness of 1.6 to 7.0 dtex and a porosity of 80 to 98%.

(Function and Effect)

When the fineness and porosity of the middle sheet are within the ranges, the super absorbent polymer particles can easily enter the fiber gap of the middle sheet before absorption of the excretion liquid and during absorption of the excretion liquid while ensuring the elasticity of the middle sheet as much as possible. Therefore, during absorption, the fibers of the middle sheet spreading in the concaves ensure a liquid passage to the individual super absorbent polymer particles. Thus, even after the super absorbent polymer particles start to swell, a decrease in diffusibility is suppressed, and gel blocking is less likely to occur.

<Sixth Aspect>

The absorbent article according to any one of the first to fifth aspects, in which a ratio of particles having a particle size of more than 500 $\mu$m is 30% by weight or less, a ratio of particles having a particle size of 500 $\mu$m or less and more than 180 $\mu$m is 60% by weight or more, a ratio of particles having a particle size of more than 106 $\mu$m and 180 $\mu$m or less is 10% by weight or less, and a ratio of particles having a particle size of 106 $\mu$m or less is 10% by weight or less in the super absorbent polymer particles.

(Function and Effect)

The super absorbent polymer particles are not particularly limited as long as the super absorbent polymer particles are commonly used in this technical field. However, those having the particle size distribution according to the present aspect are preferable.

Advantage Effects of Invention

According to the invention, there is an advantage that the absorption rate in the absorbent article including the cell absorber is improved.

Brief Description of Drawings

Fig. 1 is a plan view illustrating the inner surface of a tape-type disposable diaper in a state where a diaper is spread.
Fig. 2 is a plan view illustrating the external surface of a tape-type disposable diaper in a state where a diaper is spread.
Fig. 3 is a cross-sectional view taken along line 6-6 in Fig. 1.
Fig. 4 is a cross-sectional view taken along line 7-7 in Fig. 1.
Fig. 5(a) is a cross-sectional view taken along line 8-8 in Fig. 1, and Fig. 5(b) is a cross-sectional view taken along line 9-9 in Fig. 1.

Fig. 6 is a cross-sectional view taken along line 5-5 in Fig. 1.

Fig. 7(a) is a fragmentary plan view of a main part of an absorber, and Fig. 7(b) is a cross-sectional view taken along line 1-1 in Fig. 7(a).

Fig. 8 is a plan view of an absorber.

Fig. 9 is a plan view of an absorber.

Fig. 10 is cross-sectional views taken along line 2-2 in Figs. 8 and 9.

Fig. 11 is a plan view of an absorber illustrating bonded portions similar to Fig. 8 in a simplified manner.

Fig. 12 is a plan view of an absorber illustrating bonded portions in a simplified manner.

Fig. 13 is a plan view of an absorber illustrating bonded portions in a simplified manner.

Fig. 14 is a plan view of an absorber illustrating bonded portions in a simplified manner.

Fig. 15 is a plan view of an absorber illustrating bonded portions in a simplified manner.

Fig. 16 is a cross-sectional view taken along line 3-3 in Fig. 15.

Fig. 17 is a plan view of an absorber illustrating bonded portions in a simplified manner.

Fig. 18 is a plan view of an absorber illustrating bonded portions in a simplified manner.

Fig. 19 is a schematic plan view illustrating various arrangement examples of cells.

Fig. 20 is a cross-sectional view of various cell absorbers.

Fig. 21 is a cross-sectional view of various cell absorbers.

Fig. 22 is a schematic view illustrating an apparatus for manufacturing a cell absorber.

Fig. 23 is an explanatory diagram of a test piece in a peel strength measurement test.

Fig. 24 is an explanatory diagram of a peel strength measurement test.

Description of Embodiments

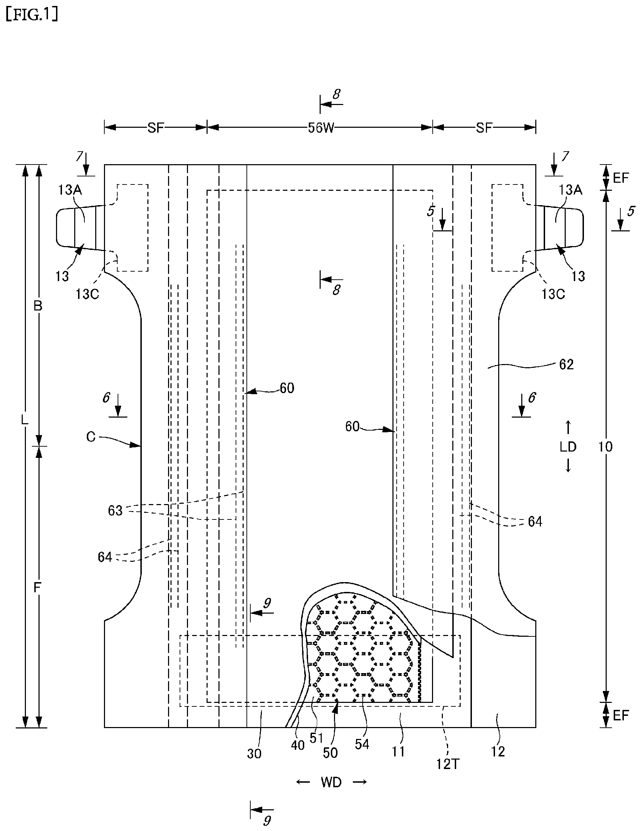

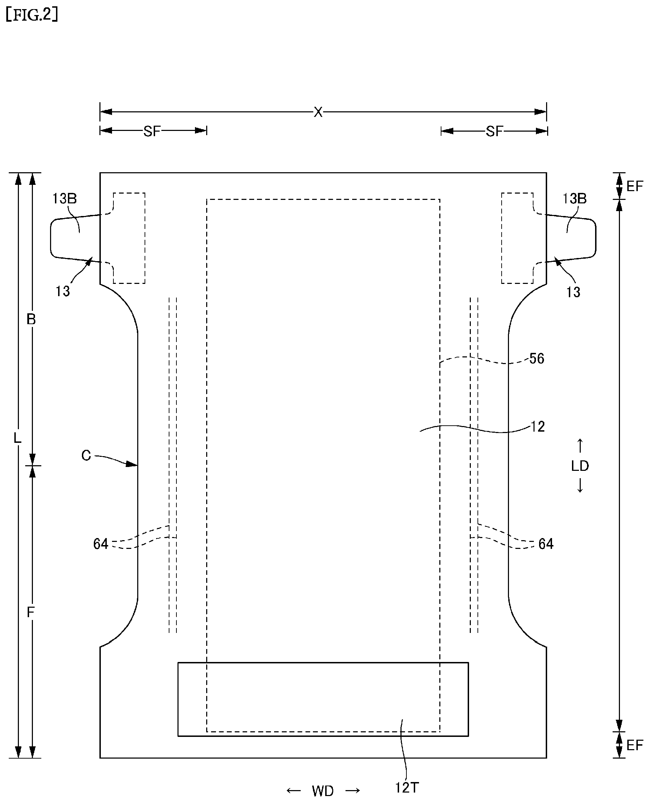

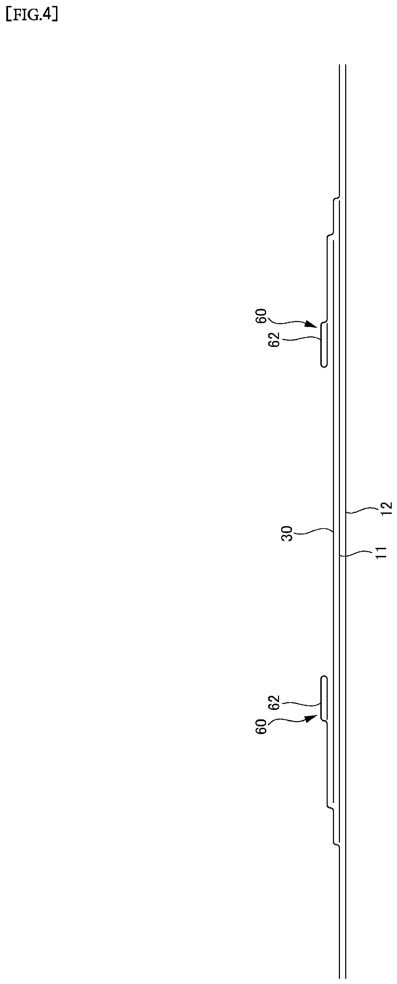

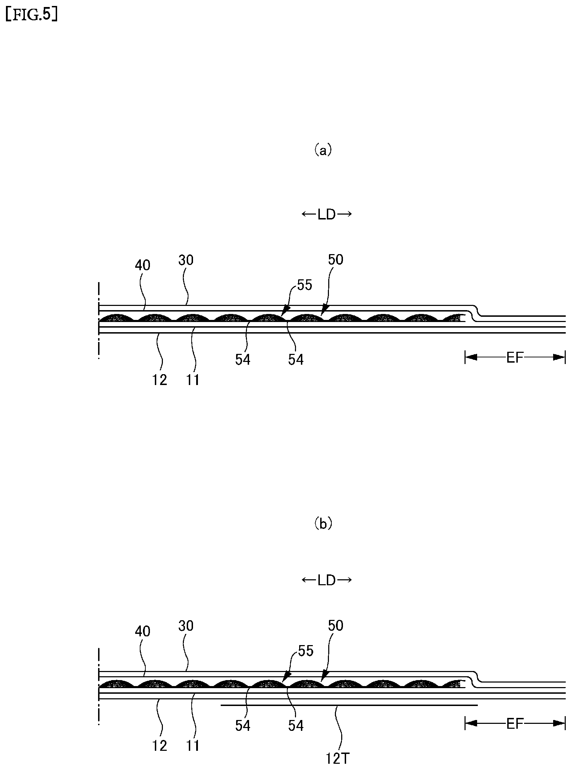

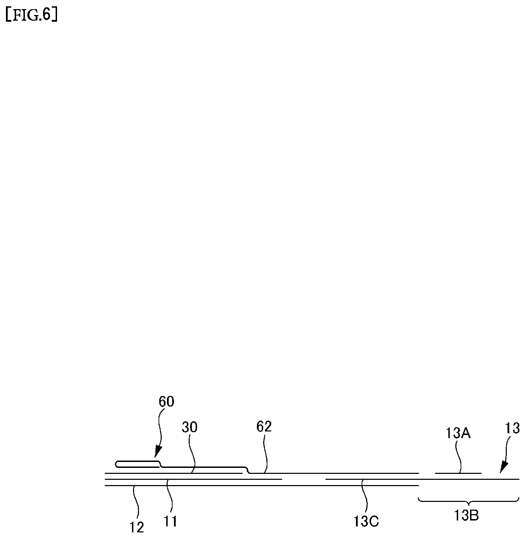

Hereinafter, a tape-type disposable diaper will be described as an example of an absorbent article with reference to the accompanying drawings. Figs. 1 to 6 illustrate examples of a tape-type disposable diaper, in which the reference sign X indicates the maximum width of the diaper excluding a fastening tape, and the reference sign L indicates the maximum length of the diaper. Each component member is fixed or bonded in the same manner as known diapers as necessary except for the fixing or bonded portion described below. As a unit for fixing or bonding, a hot melt adhesive or welding (heat welding, ultrasonic welding) can be selected as appropriate. As an application method of the hot melt adhesive, in addition to an application method such as a solid, bead, curtain, small spiral, or spiral application, it is possible to appropriately adopt a method of application to an outer peripheral surface of an elastic member such as a comb gun or wrap application.

This tape type disposable diaper has a basic structure in which an absorber 50 is interposed between a top sheet having a liquid pervious property and a liquid impervious sheet positioned on the back surface side. The tape type disposable diaper includes end flap portions EF and a pair of side flap portions SF. The end flap portions EF are portions extending to the front side and the back side of the absorber 50 respectively and do not include the absorber 50. The pair of the side flap portions SF extends laterally from the side edges of the absorber 50. In each of the side flap portions SF in a dorsal side portion B, a fastening tape 13 is provided. When a user wears the diaper, the fastening tape 13 is engaged at an appropriate place on the external surface of the ventral side portion F in a state in which the side flap portion SF of the dorsal side portion B is overlaid on the external side of the side flap portion SF of the ventral side portion F.

In addition, in this tape type disposable diaper, the entire external surface other than the fastening tape 13 is formed by an outer sheet 12. Particularly, in a region including the absorber 50, a liquid impervious sheet 11 is fixed to the internal surface side of the outer sheet 12 with an adhesive such as a hot melt adhesive. Further, the absorber 50, an intermediate sheet 40, and a top sheet 30 are stacked in this order on the internal surface side of the liquid impervious sheet 11. In the illustrated example, the top sheet 30 and the liquid impervious sheet 11 are rectangular in shape and have somewhat larger sizes in the front-back direction LD and the width direction WD than the absorber 50. The peripheral edge portion protruding from the side edges of the absorber 50 in the top sheet 30 and peripheral edge portions protruding from the side edges of the absorber 50 in the liquid impervious sheet 11 are bonded by a hot melt adhesive or the like. Further, the liquid impervious sheet 11 is formed to be slightly wider than the top sheet 30.

On both sides of an absorbent main unit section 10, three-dimensional gathers 60 rising to the skin side of a wearer are provided, and gather sheets 62 forming the three-dimensional gathers 60 are fixed in ranges from the upper both sides of the top sheet 30 to the inner surfaces of the side flap portions SF.

Details of each part will be described in order below.

(Outer Sheet)

The outer sheet 12 is a sheet constituting the external surface of a product. The outer sheet 12 has a shape in which the intermediate portions in the front-back direction LD on both the side portions are narrowed, and these sites surround the wearer's legs. A nonwoven fabric is suitable for the outer sheet 12, but it is not limited thereto. The type of the nonwoven fabric is not particularly limited. As a raw material fiber, for example, in addition to synthetic fibers such as olefin type such as polyethylene or polypropylene, polyester type, and polyamide type, regenerated fibers such as rayon and cupra, and natural fibers such as cotton can be used. As a processing method, a spun lace method, a spun bond method, a thermal bond method, an air through method, a needle punch method, and the like can be used. However, a long-fiber nonwoven fabric such as a spunbonded nonwoven fabric, an SMS nonwoven fabric, and an SMMS nonwoven fabric is preferable in that good texture and strength can be compatible. In addition to using a single piece of nonwoven fabric, it is also possible to use multiple nonwoven fabrics in layers. In the latter case, it is preferable that the nonwoven fabrics are adhered to each other with a hot melt adhesive or the like. When a nonwoven fabric is used, the basis weight of the fiber is desirably 10 to 50 g/m$^2$, particularly desirably 15 to 30 g/m$^2$. The outer sheet 12 can be omitted, and in that case, the liquid impervious sheet 11 can have the same shape as that of the outer sheet 12, such that the external surface of a product can be formed.

(Liquid Impervious Sheet)

Although the material of the liquid impervious sheet 11 is not particularly limited, for example, an olefin resin such as polyethylene or polypropylene, a laminated nonwoven fabric obtained by stacking a nonwoven fabric on a polyethylene sheet or the like, a nonwoven fabric in which liquid impermeability is substantially secured through a water proof film (in this case, a liquid impervious sheet is formed by the waterproof film and the nonwoven fabric) can be exemplified. Obviously, besides this, in recent years, liquid impervious and moisture permeable materials which have been favorably used from the standpoint of prevention of stuffiness can also be exemplified. As a sheet of this liquid-impervious and moisture-permeable material, for example, a microporous sheet can be exemplified which is obtained by kneading an olefin resin such as polyethylene or polypropylene and an inorganic filler, forming a sheet with the kneaded materials and monoaxially or biaxially stretching the sheet. Further, nonwoven fabrics using micro denier fibers and a liquid impervious sheet that is not using a water proof film can also be used as the liquid impervious sheet 11. The liquid impervious sheet includes the sheet obtained by high leak proof treatment by heating or applying pressure to reduce air gaps of fibers, and the sheet obtained by applying a superabsorbent resin, a hydrophobic resin, or a water repellent agent.

(Top Sheet)

As the top sheet 30, a porous or non-porous nonwoven fabric having liquid permeability can be used. The type of constituent fibers of the nonwoven fabric is not particularly limited. Examples of the constituent fibers can include synthetic fibers such as olefin type such as polyethylene and polypropylene, polyester type, and polyamide type, regenerated fibers such as rayon and cupra, natural fibers such as cotton, mixed fibers and conjugate fibers in which two or more of these are used, and the like. Further, the nonwoven fabric may be manufactured by any processing. Examples of processing methods can include known methods such as a spun lace method, a spun bond method, a thermal bond method, a melt blown method, a needle punch method, an air through method, and a point bond method. For example, the spun lace method is preferable when flexibility and drapeability are required, and the thermal bond method is preferable when bulkiness and softness are required.

(Intermediate Sheet)

The intermediate sheet 40 is bonded to the back surface of the top sheet 30 to promptly move excretion liquid passing through the top sheet 30 to the side of the absorber 50 and to prevent returning. For bonding between the intermediate sheet 40 and the top sheet 30, heat embossing or ultrasonic welding can be used in addition to using a hot melt adhesive. As the intermediate sheet 40, a resin film having a large number of through holes can be used in addition to using a nonwoven fabric. As the nonwoven fabric, a material similar to that described in the section of the top sheet 30 can be used. However, the material having a higher hydrophilicity than that of the top sheet 30 or the material having a high fiber density is preferable since those have excellent liquid transfer characteristics from the top sheet 30 to the intermediate sheet 40.

Although the intermediate sheet 40 in the illustrated embodiment is shorter than the width of the absorber 50 and disposed at the center portion, it may be provided over the maximum width. The length of the intermediate sheet 40 in the front-back direction LD may be the same as the maximum length of a diaper, may be the same as the length of the absorber 50, or may be within a short length range around a region receiving a liquid.

(Three-dimensional Gather)

To prevent lateral movement of excrement on the top sheet 30 and to prevent lateral leakage, it is preferable to provide the three-dimensional gathers 60 projecting (standing) from the inner faces on both the sides of a product in the width direction WD.

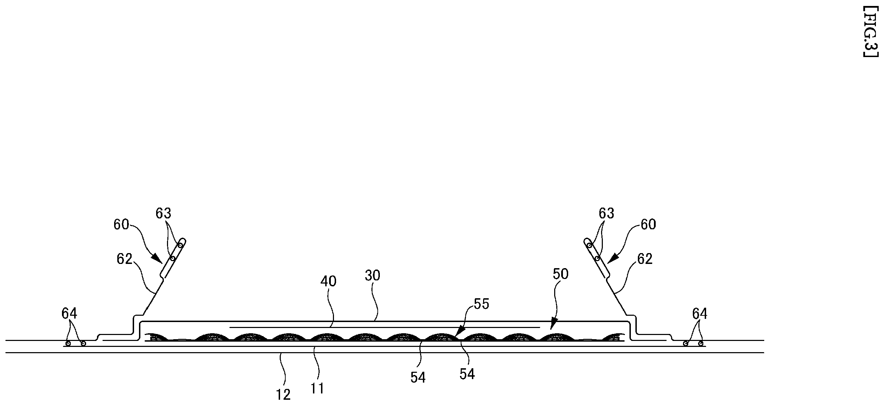

Each three-dimensional gather 60 is composed of a gather sheet 62 and an elongated gather elastic member 63 fixed to the gather sheet 62 in a stretched state along the front-back direction LD. As this gather sheet 62, a water repellent nonwoven fabric can be used, and rubber thread or the like can be used as the elastic member 63. As illustrated in Figs. 1 and 3, a plurality of the elastic members may be provided on each side, or only one elastic member may be provided on each side.

The inner surface of the gather sheet 62 has a fixed start point in the width direction WD on the side portion of the top sheet 30. A portion outside in the width direction WD from this fixed start point is fixed with a hot melt adhesive or the like to the side portion of the liquid impervious sheet 11 and the side portion of the outer sheet 12 positioned at the outside portion.

In the periphery of the leg, the inside in the width direction WD from the fixed start point of each three-dimensional gather 60 is fixed on the top sheet 30 at both ends of the product in the front-back direction LD. However, since the portion therebetween is a non-fixed free portion, this free portion is erected by contraction force of one or plurality of the elastic members 63. Since a diaper is attached to the body in a boat shape in wearing the diaper, and the contraction force of one or plurality of the elastic members 63 acts, the three-dimensional gathers 60 erect by the contraction force of one or plurality of the elastic members 63 and come in close contact with the legs. As a result, so-called lateral leakage from around the legs is prevented.

Unlike the illustrated example, both end portions in the front-back direction LD in the portion of the inside in the width direction WD of each gather sheet 62 are fixed in a state folded in two having a base end side portion, which extends inward from a portion outside in the width direction WD and a tip side portion, which is folded back on the body side from the end edge on the center side in the width direction WD of the base end side portion and extending outward in the width direction WD, and the portion therebetween may be a non-fixed free portion.

(Flat Gather)

As illustrated in Figs. 1 to 3, in each side flap portion SF, on the outside in the width direction WD in the vicinity of the fixed start point of the fixed portion of the gather sheet 62, between the gather sheet 62 and the liquid impervious sheet 11, the elastic members 64, which are made of elongated elastic members such as rubber threads, around the leg portions are fixed in a state stretching along the front-back direction LD, whereby the leg portion of each side flap portion SF is formed as a flat gather. The elastic members 64 around each leg portion can also be disposed between the liquid impervious sheet 11 and the outer sheet 12 in the side flap portion SF. As in the illustrated example, a plurality of elastic members 64 around the leg portions may be provided on each side, or only one elastic member 64 may be provided on each side.

(Fastening Tape)

As illustrated in Figs. 1, 2, and 6, each fastening tape 13 includes a sheet base material forming a tape attaching portion 13C fixed to the side portion of a diaper and a tape main unit section 13B projecting from the tape attaching portion 13C, and an engagement portion 13A with respect to the ventral side, which is provided in the intermediate portion in the width direction WD of the tape main unit section 13B in the seat base material. A tip end side from the engagement portion 13A is a tab part. The tape attaching portion 13C of the fastening tape 13 is sandwiched between the gather sheet 62 forming an inner layer in the side flap portion and the outer sheet 12 forming an outer layer. The tape attaching portion 13C is adhered to both the sheets 62 and 12 with a hot melt adhesive. In addition, the engagement portion 13A is bonded to the sheet base material with an adhesive so that it cannot be removed.

A hook member (male member) of a mechanical fastener (hook and loop fastener) is suitable as the engagement portion 13A. The hook member has a large number of engagement projections on its external surface side. The engagement projection has a check mark shape, a J shape, a mushroom shape, a T shape, and a double J shape (a shape bonded back to back of a J shape), but may have any shape. Obviously, a pressure sensitive adhesion material layer can also be provided as an engagement portion of the fastening tape 13.

Further, as the sheet base material forming from the tape attaching portion to the tape main unit section, in addition to various nonwoven fabrics such as a spunbonded nonwoven fabric, an air through nonwoven fabric, and a spunlace nonwoven fabric, a plastic film, a polyethylene laminated nonwoven fabric, paper, or a composite material thereof can be used.

(Target Sheet)

It is preferable to provide a target sheet 12T having a target for facilitating engagement at the engagement portion of each fastening tape 13 in the ventral side portion F. In a case where the engagement portion 13A is the hook member, there can be used the target sheet 12T having a large number of loops made of threads to which engagement projections of the hook member are tangled and which is provided on a surface of the sheet base member made of a plastic film or a nonwoven fabric. Further, in the case of an adhesive layer, it is possible to use a sheet base material made of a plastic film having a smooth surface with high adhesiveness and subjected to a release treatment. In addition, in a case where the engagement portion of the fastening tape 13 in the ventral side portion F is made of a nonwoven fabric, for example, when the outer sheet 12 in the illustrated example is made of a nonwoven fabric, and the engagement portion 13A of the fastening tape 13 is the hook member, the target sheet 12T may be omitted, and the hook member can be entangled and engaged with the nonwoven fabric of the outer sheet 12. In this case, the target sheet 12T may be provided between the outer sheet 12 and the liquid impervious sheet 11.

(Absorber)

The absorber 50 is a part that absorbs and retains liquid content of excrement. The absorber 50 can be adhered to the components on at least one of the front surface side and back surfaces side via an adhesive such as a hot melt adhesive.

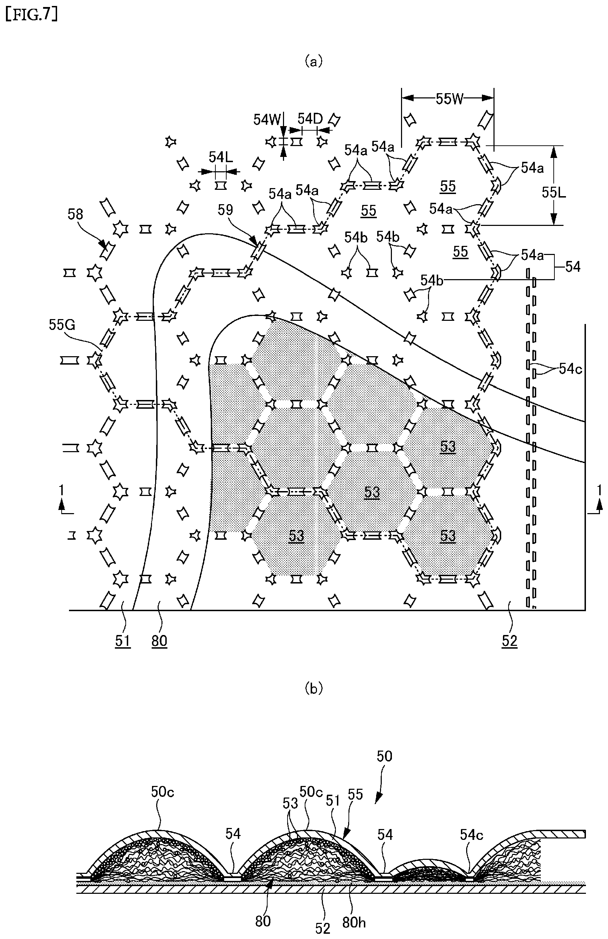

As enlarged and illustrated in Fig. 7, the absorber 50 is a cell absorber including the upper sheet 51, the lower sheet 52 disposed on the back surface side of the upper sheet, cells (small chambers) 55 which are surrounded by the bonded portions 54 of the upper sheet 51 and the lower sheet 52 and at which the upper sheet 51 and the lower sheet 52 are not bonded, and super absorbent polymer particles 53 contained in the cells 55. As described above, by distributing and holding the super absorbent polymer particles 53 in the large number of the cells 55 whose entire peripheries are surrounded by the bonded portions 54, it is possible to prevent uneven distribution of the super absorbent polymer particles 53 in the absorber 50.

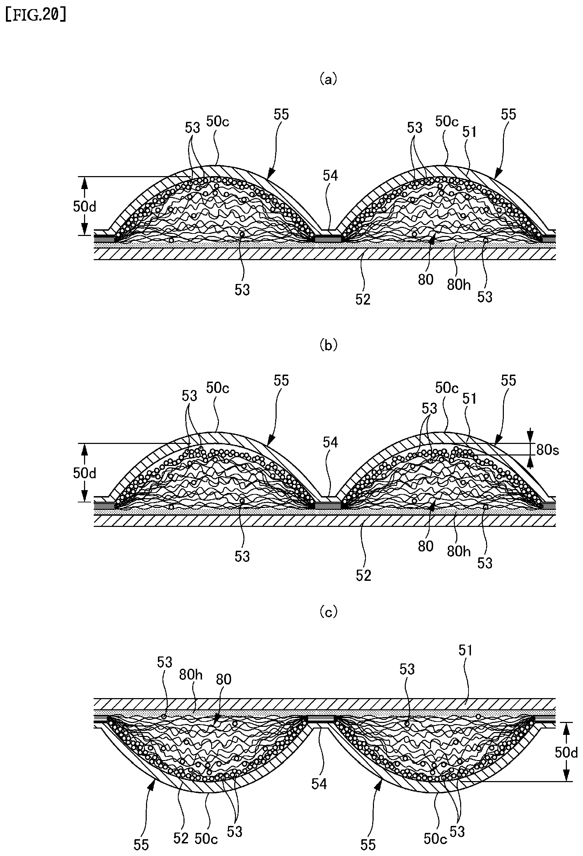

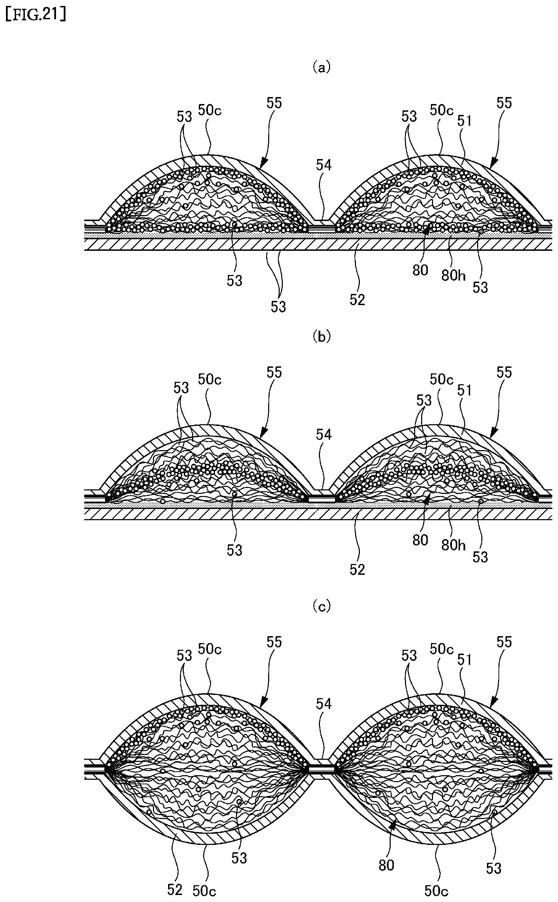

To facilitate arrangement of the super absorbent polymer particles 53 at the time of production, and to ensure the volume after absorption and swelling, the upper sheet 51 in the cells 55 corresponds to concaves 50c depressed to the outsides of the cells 55 in the spread state, i.e. the concaves 50c are formed in parts of the upper sheet 51 included in the respective cells 55. However, additionally as in an example illustrated in Fig. 21(c), the concaves 50c may be formed in parts of the lower sheet 52 included in the respective cells 55. Further disclosed is an example as illustrated in Fig. 20(c), in which the concaves 50c may alternatively be formed in parts of the lower sheet 52 included in the respective cells 55. A depth 50d of the concaves 50c is not particularly limited, and is preferably 2 to 10 mm, particularly preferably about 8 to 10 mm. The concaves 50c can be formed by embossing as described later.

As illustrated in Fig. 7(b) and Fig. 20(a), a middle sheet 80 made of a nonwoven fabric is interposed between the upper sheet 51 and the lower sheet 52. Therefore, the three layers of the upper sheet 51, the middle sheet 80, and the lower sheet 52 are bonded at the bonded portions 54. Further, the middle sheet 80 is compressed in a thickness direction at the bonded portions 54 and expands to the inside of the concaves 50c in a part located in the cell 55 (in other words, the fiber density decreases as the distance from the bonded portions increases). In this way, the concaves 50c are less likely to be crushed by a pressure applied in a package state of a product or a pressure applied during wearing. Even when the concaves 50c are crushed, due to the elasticity of the middle sheet 80, restoration of shapes of the concaves 50c is promoted to at least a part where the middle sheet 80 enters or a volume close thereto. Further, during absorption of the excretion liquid, the super absorbent polymer expands the fiber gap and can swell while entering the gap, easily compressing the middle sheet 80, or entering the gap and compressing the middle sheet 80, and thus the presence of the middle sheet 80 is unlikely to hinder swelling of the super absorbent polymer particles 53. Further, the fibers of the middle sheet 80 spreading in the concaves 50c ensure a liquid passage to the individual super absorbent polymer particles 53. Thus, even after the super absorbent polymer particles 53 start to swell, a decrease in diffusibility is suppressed, and gel blocking is less likely to occur. Therefore, by the synergistic action thereof, the absorption rate of the disposable diaper including the present cells 55 absorber 50 (particularly, an initial stage of absorption) is improved.

The upper sheet 51 is not particularly limited as long as the upper sheet 51 is a liquid pervious material. Similarly to the top sheet 30, a porous or non-porous nonwoven fabric or a porous plastic sheet can be used for the upper sheet 51. In the case of using a nonwoven fabric for the upper sheet 51, as the constituent fibers, for example, synthetic fibers (including not only single component fibers but also conjugate fibers) such as olefin type such as polyethylene or polypropylene, polyester type, and polyamide type, regenerated fibers such as rayon and cupra, and natural fibers such as cotton and pulp can be selected without limitation, and it is preferable to use a thermoplastic resin fiber because of excellent thermal processability. A fiber stacking method for the nonwoven fabric can be used irrespective of a wet type or a dry type. Since the upper sheet 51 affects the absorption rate, hydrophilic fibers, particularly dry nonwoven fabrics using natural fibers such as cotton and pulp as a raw material, more particularly air-laid pulp nonwoven fabrics of 70% by weight or more of pulp (remaining amount in the case of less than 100% by weight may be occupied by appropriate synthetic fibers) are particularly suitable for the upper sheet 51. The fiber bonding method of the nonwoven fabric is not particularly limited, but to prevent the super absorbent polymer particles 53 from being detached, it is preferable to use a bonding method which increases fiber density, such as a spun bond method, a melt blown method, and a needle punch method. In addition, the fineness, basis weight, and thickness of the nonwoven fabric are preferably about 2.0 to 7.0 dtex, about 18 to 50 g/m$^2$, and about 0.10 to 0.60 mm, respectively. In the case of using a porous plastic sheet, its pore size is preferably smaller than the outer shape of the super absorbent polymer particles 53 to prevent the super absorbent polymer particles 53 from falling off through the sheet. Further, when the material of the upper sheet 51 is hydrophobic, a hydrophilic agent can also be contained.

The lower sheet 52 may be formed of the same material as that of the upper sheet 51. However, it is possible to adopt a liquid impervious material. The liquid impervious material usable for the lower sheet 52 can be appropriately selected and used from the materials described in the section of the liquid impervious sheet 11. Although not illustrated, the upper sheet 51 and the lower sheet 52 may be one side layer and another side layer in which one sheet of material is folded in two.

The middle sheet 80 is not particularly limited as long as the middle sheet 80 is a nonwoven fabric. However, the fineness of the constituent fibers of the nonwoven fabric is preferably about 1.6 to 7.0 dtex, and more preferably 5.6 to 6.6 dtex. Further, the porosity of the nonwoven fabric of the middle sheet 80 is preferably 80 to 98%, and more preferably 90 to 95%. When the fineness and porosity of the middle sheet 80 are within these ranges, the super absorbent polymer particles 53 can easily enter the fiber gap of the middle sheet 80 before absorption of the excretion liquid and during absorption of the excretion liquid while ensuring the elasticity of the middle sheet 80 as much as possible. Therefore, during absorption, the fibers of the middle sheet 80 spreading in the concaves 50c ensure a liquid passage to the individual super absorbent polymer particles 53. Thus, even after the super absorbent polymer particles 53 start to swell, a decrease in diffusibility is suppressed, and gel blocking is less likely to occur. The thickness of the middle sheet 80 may be appropriately determined in consideration of the depth 50d of the concaves 50c, the degree of penetration into the concaves 50c, etc. The thickness is preferably 10% to 90% of the depth 50d of the concaves 50c, and more preferably 70% to 90% thereof. The basis weight of the middle sheet 80 may be appropriately determined for the same reason, and is preferably about 25 to 40 g/m$^2$ in the above thickness range. As the constituent fibers of the nonwoven fabric used for the middle sheet 80, for example, synthetic fibers (including not only single component fibers but also conjugate fibers such as core sheath) such as olefin type such as polyethylene or polypropylene, polyester type, and polyamide type, regenerated fibers such as rayon and cupra, and natural fibers such as cotton can be selected without limitation, and a combination thereof can be used. In order to increase the porosity of the nonwoven fabric of the middle sheet 80 (to increase the fiber gap), it is preferable that the constituent fibers are crimped fibers. Further, when the constituent fibers of the nonwoven fabric of the middle sheet 80 are hydrophilic fibers (including hydrophobic fibers becoming hydrophilic by a hydrophilizing agent), the water retention is increased. When the constituent fibers are hydrophobic fibers, the diffusibility is improved. A fiber bonding method of the nonwoven fabric is not particularly limited. However, to sufficiently bind the fibers to ensure elasticity while increasing the porosity (widening the fiber gap), an air through nonwoven fabric in which fibers are bound by hot air heating is preferable for the middle sheet 80.

As long as a surface of the middle sheet 80 facing the concaves 50c enters the concaves 50c, as illustrated in Figs. 20(a) and 20(c) and Figs. 21(a) to 21(c), the surface may come into contact with inner surfaces of the concaves 50c. However, the surface may alternatively be separated therefrom as illustrated in Fig. 20(b). When the surface of the middle sheet 80 facing the concaves 50c is separated from the inner surfaces of the concaves 50c, a separation distance 80s can be determined as appropriate, and is 30% or less of the depth 50d of the concaves 50c.

The middle sheet 80 may adhere to at least one of the upper sheet 51 and the lower sheet 52 by a hot melt adhesive 80h both in the cells 55 and at the bonded portions 54 as illustrated in Figs. 20(a) to 20(c) and Figs. 21(a) and 21(b), or may not adhere to both the upper sheet 51 and the lower sheet 52 as illustrated in Fig. 21(c).

It is preferable that almost all (for example, 95% or more) of the super absorbent polymer particles 53 are not fixed to the upper sheet 51, the lower sheet 52, and the middle sheet 80, and are freely movable. However, a part or almost all (for example, 95% or more) of the super absorbent polymer particles 53 can be bonded or adhered to at least one of the upper sheet 51, the lower sheet 52, and the middle sheet 80. Further, the super absorbent polymer particles 53 may be agglomerated to some extent. In particular, in a case where the super absorbent polymer particles 53 are freely movable in the cells 55, when the cells 55 have hollow portions, the super absorbent polymer particles 53 move in the cells 55 during use. Thus, there is concern that noise may be generated or absorption may be inhibited due to uneven distribution of the super absorbent polymer particles 53 in the cells 55. Therefore, to solve this problem, it is one preferable mode to bring the surface of the middle sheet 80 facing the concaves 50c into contact with the inner surfaces of the concaves 50c as described above, in other words, to fill almost the entire cells 55 including the concaves 50c with fibers of the middle sheet 80 having a high porosity. In this way, the super absorbent polymer particles 53 are captured by the fibers of the middle sheet 80, or pressed against the upper sheet 51 or the lower sheet 52, or captured and pressed, so that free movement hardly occurs. Therefore, while preventing inhibition of swelling of the super absorbent polymer particles 53, it is possible to prevent generation of noise due to movement of the super absorbent polymer particles 53 and inhibition of absorption due to uneven distribution of the super absorbent polymer particles 53 in the cells 55.

The super absorbent polymer particles 53 are most present on an upper surface of the middle sheet 80 and decrease toward the lower side as illustrated in Figs. 20(a) and 20(b) and Fig. 21(c), so that scratchy feeling (uncomfortable feeling) of the super absorbent polymer particles 53 is rarely transmitted to a hand due to intervention of the middle sheet 80 when the user touches an outer surface of the diaper, which is preferable. In particular, when the middle sheet 80 is a bulky nonwoven fabric having a high porosity, since the super absorbent polymer particles 53 can enter the fiber gap of the middle sheet 80 before absorption of the excretion liquid and during absorption of the excretion liquid, the absorption rate is further improved. That is, in an initial stage of the absorption, the absorption proceeds on the upper surface of the middle sheet 80 where a large number of the super absorbent polymer particles 53 are distributed, and a rate there of is limited. Therefore, in the initial stage of the absorption, a large amount of excretion liquid penetrates into the middle sheet 80 having a small number of the super absorbent polymer particles 53, and absorbed by the super absorbent polymer particles 53 in the middle sheet 80, temporarily stored until absorbed by the super absorbent polymer particles 53, or diffuse to the surrounding cells 55. The excretion liquid diffused to the surroundings is absorbed by the super absorbent polymer particles 53 in the middle sheet 80 existing there or sucked up by the super absorbent polymer particles 53 abundant thereabove. Then, in a process in which each of the super absorbent polymer particles 53 absorbs the excretion liquid, the super absorbent polymer expands the fiber gap and swells while penetrating into the gap or compressing the middle sheet 80. Due to such an absorption mechanism, the excretion liquid rapidly spreads over a wide area of the cell 55 absorber 50 and is in a state of being received inside the cell 55 absorber 50. Thus, not only the absorption rate is improved, but also a reflowing prevention property is excellent. In addition, in order to favorably exhibit such an absorption mechanism, the concaves 50c are formed at least in a part of each cell 55 in the upper sheet 51.

A degree of distribution of the super absorbent polymer particles 53 in the cell 55 can be determined as appropriate. In a normal case, a weight ratio of the super absorbent polymer particles 53 present on the upper surface of the middle sheet 80 is preferably 50% or more of the total amount, and a weight ratio of the super absorbent polymer held in the middle sheet 80 (that is, not on the lower sheet 52) is preferably 45% or more of the total amount.

Naturally, the distribution of the super absorbent polymer particles 53 in the cell 55 is not limited thereto. Therefore, as illustrated in Fig. 20(c), it is possible to adopt a distribution in which the super absorbent polymer particles 53 are most present on the upper surface of the lower sheet 52 and decrease upward therefrom. Alternatively, as illustrated in Fig. 21(a), it is possible to adopt a distribution in which the number of super absorbent polymer particles 53 present on the upper surface of the middle sheet 80 and the upper surface of the lower sheet 52 is larger than that in a part therebetween. Further, as illustrated in Fig. 21(b), it is possible to adopt a distribution in which the super absorbent polymer particles 53 are most present in the middle of the middle sheet 80 in the thickness direction and decrease upward and downward therefrom. This mode can be formed by forming the middle sheet 80 into a two-layer nonwoven fabric and interposing the super absorbent polymer particles 53 between the layers.

As the super absorbent polymer particles 53, those used for this type of absorbent articles can be used on an as-is basis. A particle size of the super absorbent polymer particles is not particularly limited. For example, it is preferable that a ratio of particles having a particle size of more than 500 $\mu$m is 30% by weight or less, a ratio of particles having a particle size of 500 $\mu$m or less and more than 180 $\mu$m is 60% by weight or more, a ratio of particles having a particle size of more than 106 $\mu$m and 180 $\mu$m or less is 10% by weight or less, and a ratio of particles having a particle size of 106 $\mu$m or less is 10% by weight or less. Note that measurement of the particle size is performed as follows. That is, standard sieves (JIS Z8801-1:2006) of 500 $\mu$m, 180 $\mu$m, and 106 $\mu$m and a saucer are disposed in this order from the top, 10 g of a sample of super absorbent polymer particles is put into the sieve of 500 $\mu$m at the top, sieving is performed (5 minutes shaking), and then the weight of particles remaining on each sieve is measured. As a result of this sieving, weight ratios with respect to input amounts of the samples remaining on the respective sieves of 500 $\mu$m, 180 $\mu$m, and 106 $\mu$m and the sample remaining on the saucer are set to the ratio of particles of more than 500 $\mu$m, the ratio of particles of 500 $\mu$m or less and more than 180 $\mu$m, the ratio of particles of more than 106 $\mu$m and 180 $\mu$m or less, and the ratio of particles of 106 $\mu$m or less, respectively.

The material of the super absorbent polymer particles 53 can be used without particular limitation, but the material having the water absorption capacity of 40 g/g or more is suitable. As the super absorbent polymer particles 53, starch-based, cellulose-based, and synthetic polymerbased, and starch-acrylic acid (salt) graft copolymers, saponified starch-acrylonitrile copolymers, sodium carboxymethyl cellulose crosslinked products, acrylic acid (salt) polymers, and the like can be used. As the shape of the super absorbent polymer particles 53, the shapes of particulate materials which are usually used are suitable, but other shapes can also be used.

The super absorbent polymer particles 53 having a water absorption rate of 70 seconds or less, particularly 40 seconds or less, are suitably used. If the water absorption rate is too slow, back-flow, in which the liquid fed into the absorber 50 returns to the outside of the absorber 50, is likely to occur.

In addition, the super absorbent polymer particles 53 having the gel strength of 1,000 Pa or more are preferably used. Thereby, even when the absorber 50 is bulky, it is possible to effectively suppress stickiness after liquid absorption.

The basis weight of the super absorbent polymer particles 53 can be appropriately determined according to the absorption amount required for the use of the absorber 50. Therefore, although it cannot be said unconditionally, the basis weight can be 50 to 350 g/m$^2$. When the basis weight of the polymer is less than 50 g/m$^2$, it is difficult to secure the absorption amount. When it exceeds 350 g/m$^2$, the effect is saturated.

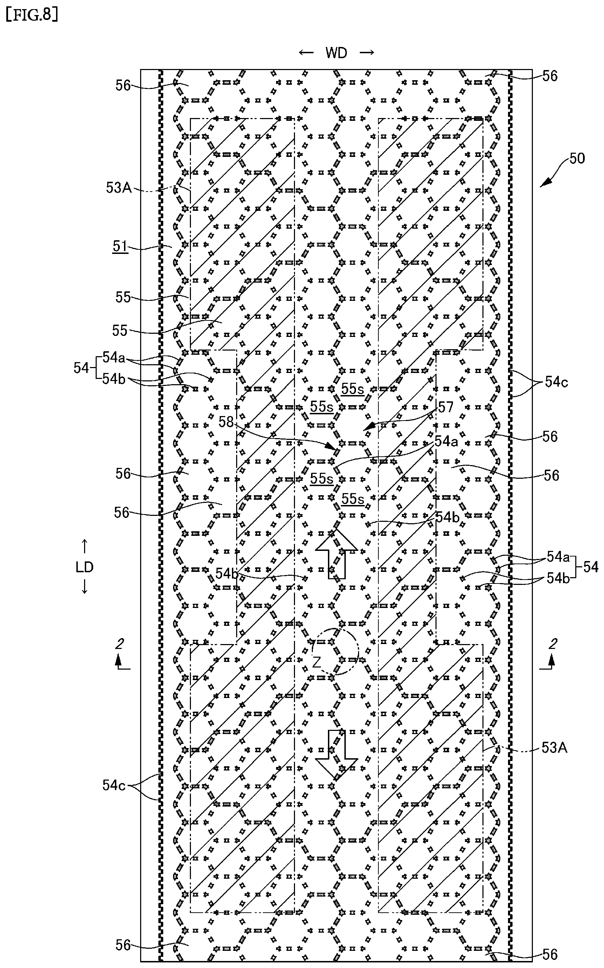

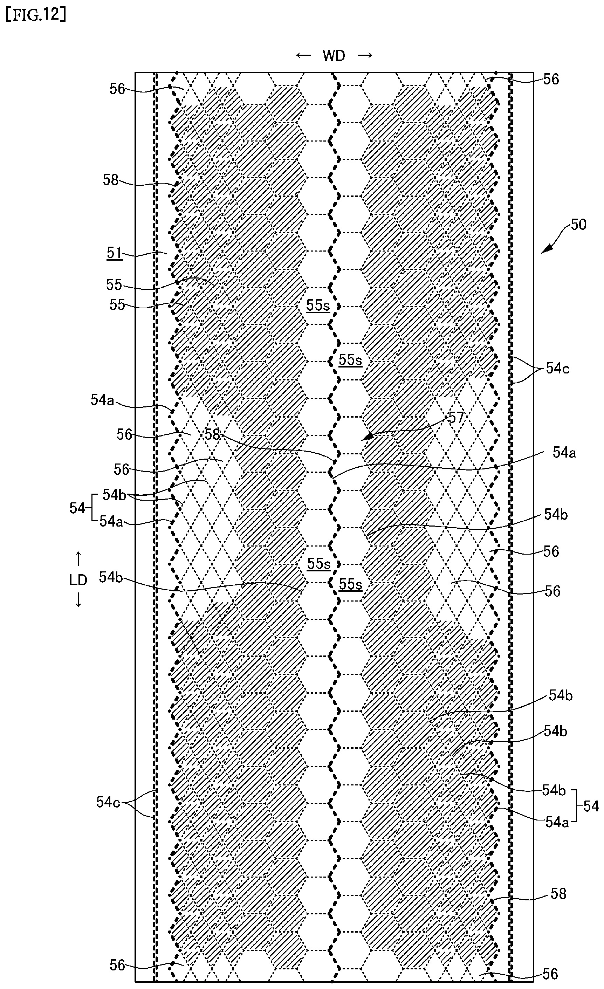

A planar shape of each of the cells 55 can be determined as appropriate, and can be set to a hexagonal shape, a rhombic shape, a square shape, a rectangular shape, a circular shape, an elliptical shape, etc. as illustrated in Fig. 8, Fig. 12, Fig. 19, etc. It is desirable to form a polygon to achieve denser arrangement, and arrangement without gaps as in the illustrated example is desirable. In addition to arranging the cells 55 having the same shape and the same dimension, the cells 55 may be arranged by combining a plurality of types of cells 55 different in at least one of the shape and dimension as illustrated in Fig. 12.

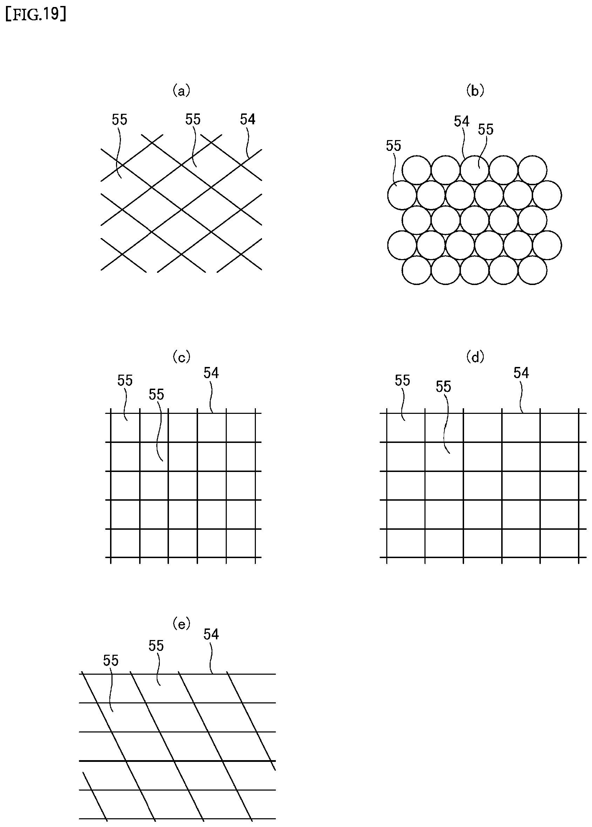

The planar arrangement of the cells 55 (that is, the same applies to an aggregated part of the super absorbent polymer particles 53) can be appropriately determined. A regularly repeated planar arrangement is preferable. In addition to a regularly repeated shape such as an oblique lattice shape illustrated in Fig. 19(a), a hexagonal lattice shape illustrated in Fig. 19(b) (these shapes are also referred to as staggered shapes), a square lattice shape illustrated in Fig. 19(c), a rectangular lattice shape illustrated in Fig. 19(d), or a parallel body lattice shape illustrated in Fig. 19(e) (a mode in which two groups of a large number of parallel oblique rows are provided to intersect with each other as illustrated in the figure) (including those inclined at an angle less than 90 degrees with respect to a stretchable direction), it is possible to adopt a shape in which a group of cells 55 (arrangement in units of groups may be regular or irregular and may correspond to a pattern, a character, etc.) is regularly repeated.

The size of each cell 55 can be appropriately determined, and for example, the length 55L of the front-back direction LD can be about 8 to 30 mm, and the length 55W of the width direction WD can be about 10 to 50 mm. The area of each cell 55 can be set to about 65 to 1,650 mm$^2$.

It is desirable that the bonded portion 54 for bonding the upper sheet 51 and the lower sheet 52 be bonded by welding the upper sheet 51 and the lower sheet 52 like ultrasonic welding or heat sealing, but it may be bonded with a hot melt adhesive.

The bonded portions 54 of the upper sheet 51 and the lower sheet 52 are disposed to surround each cell 55 and may be formed in a dotted line pattern (intermittently in a direction surrounding each cell 55) as in the illustrated example or in a continuous line shape as long as the bonded portions 54 correspond to the boundary between adjacent cells. In a case where the bonded portions 54 are formed intermittently, it is preferable that the super absorbent polymer particles 53 are not present between the bonded portions 54 in the direction surrounding each of the cells 55 or are supposed to be less than those in the cell 55 even if they are present therein. In particular, when the bonded portions are provided in the dotted line pattern (intermittently), a fiber group of the middle sheet passes between adjacent bonded portions and extends between a large number of cells. Therefore, since a liquid diffusion passage is formed between the adjacent bonded portions, the liquid diffusibility between cells is improved, so that the absorption rate is improved.

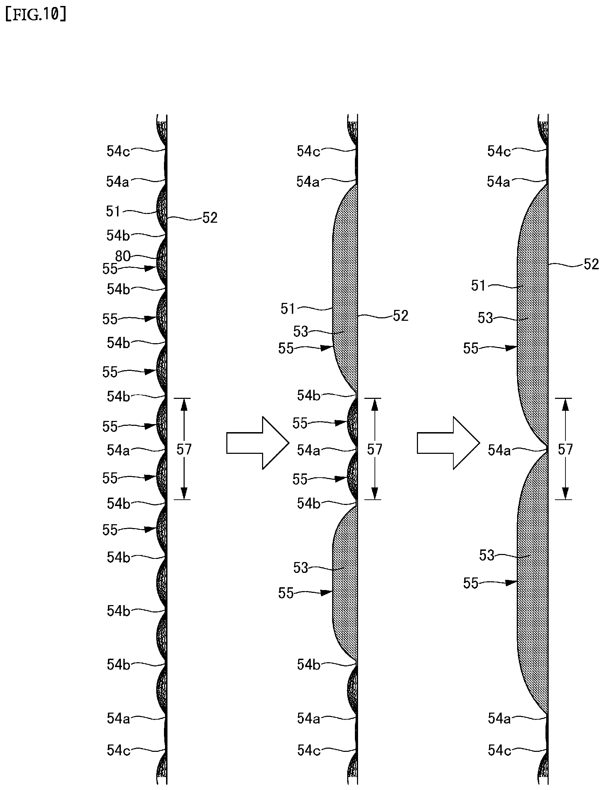

As illustrated Fig. 10, the bonded portions 54 may correspond to weak bonded portions 54b that can be peeled off by swelling forces of the super absorbent polymer particles 53 in the adjacent cells 55, or correspond to strong bonded portions 54a that do not basically peeled off by the swelling forces of the super absorbent polymer particles 53 in the adjacent cells 55. To cope with the swelling of the super absorbent polymer particles 53 having individual volumes of the cells 55 or more, it is preferable that some or all of the bonded portions 54 correspond to the weak bonded portions 54b. By having the weak bonded portions 54b, the cells 55 adjacent to each other with the weak bonded portions 54b interposed therebetween can become one large cell 55 by being peeled off and united due to absorption and swelling pressures of the super absorbent polymer particles 53 in the cells 55. Peel strengths of a facing surface between the upper sheet 51 and the middle sheet 80 and a facing surface between the middle sheet 80 and the lower sheet 52 in the weak bonded portions 54b can be determined as appropriate. However, in a normal case, the peel strengths are preferably about 0.800 N/4 cm. In addition, peel strengths of a facing surface between the upper sheet 51 and the middle sheet 80 and a facing surface between the middle sheet 80 and the lower sheet 52 in the strong bonded portions 54a can be appropriately determined. However, it is preferable that the peel strength is 1.1 times or more a higher one of the peel strengths of the facing surface between the upper sheet 51 and the middle sheet 80 and the facing surface between the middle sheet 80 and the lower sheet 52 in the weak bonded portions 54b.

However, in a case where the middle sheet 80 is provided between the upper sheet 51 and the lower sheet 52 as in the cell 55 absorber 50, when there are weak bonded portions 54b where the upper sheet 51 and the middle sheet 80 peel off first, or weak bonded portions 54b where the middle sheet 80 and the lower sheet 52 peel off first in the bonded portions 54, the shape of swelling of the cell 55 absorber 50 becomes irregular, and there is concern that swelling may be hindered or the wearing feeling may deteriorate. Therefore, in the weak bonded portions 54b, it is preferable that the peel strength of one of the facing surface between the upper sheet 51 and the middle sheet 80 and the facing surface between the middle sheet 80 and the lower sheet 52 is lower than the peel strength of the other one. In this way, peeling of the one having the weaker peel strength preferentially occurs, and swelling of the cell 55 absorber 50 becomes uniform and smooth. For example such a structure can be constructed by bonding only the facing surface between the upper sheet 51 and the middle sheet 80 or bonding only the facing surface between the middle sheet 80 and the lower sheet 52 in both the inside of the cell 55 and the bonded portions 54, thereby setting the peel strength of one of the facing surface between the upper sheet 51 and the middle sheet 80 and the facing surface between the middle sheet 80 and the lower sheet 52 in the weak bonded portions 54b not subjected to bonding to be lower than the peel strength of the other one subjected to bonding. Peel strengths of a facing surface between the upper sheet 51 and the middle sheet 80 and a facing surface between the middle sheet 80 and the lower sheet 52 in the weak bonded portions 54b can be determined as appropriate. However, in a normal case, the higher peel strength is preferably about 0.040 to 0.800 N/4 cm, and the lower peel strength is preferably 0.5 times or less the higher peel strength.

In particular, in the mode in which the super absorbent polymer particles 53 are most present on the upper surface of the middle sheet 80 and decrease downward therefrom as in the examples illustrated in Figs. 20(a) and 20(b) and Fig. 21(c), respectively, considering that the absorption mechanism is favorably exhibited, it is preferable that the peel strength of the facing surface between the upper sheet 51 and the middle sheet 80 is lower than the peel strength of the facing surface between the middle sheet 80 and the lower sheet 52.

Meanwhile, the strong bonded portions 54a are portions basically not peel off even when the cells 55 on both sides absorb and swell, and thus have effects that diffusibility is improved by continuing in a specific direction, a flow of a gelled material of the super absorbent polymer particles 53 is prevented, and the contact area on the front surface side is reduced. Therefore, by combining these portions with the weak bonded portions, it is possible to construct a cell absorber having various characteristics as described later. Note that when the bonded portions 54 positioned on the outermost side in the width direction WD are peeled off, there is concern that the super absorbent polymer particles 53 or the gelled super absorbent polymer particles 53 may leak laterally to the outside of the absorber 50, and therefore it is desirable that the bonded portions are the strong bonded portions 54a. From the same viewpoint, it is preferable that the upper sheet 51 and the lower sheet 52 are extended to the outside in the width direction WD to some extent beyond the region where the cells 55 are formed, and the edge bonded portions 54c are provided in the extended portions for the reinforcement.

The difference in bonding strength may be easily formed by changing the area of each of the bonded portions 54 but is not limited thereto. For example, in the case of forming the bonded portions 54 with a hot melt adhesive, a method in which the type of the hot melt adhesive is varied depending on the sites of the cells can be adopted. In particular, in the case of forming the bonded portions 54 by welding the upper sheet 51 and the lower sheet 52, the weak bonded portions 54b can be formed only by forming the bonded portions 54 into the dotted line pattern and widening the point interval 54D. However, since the bonded portions 54 can be formed at the boundary between the adjacent cells 55, if the point interval 54D becomes too large, the gaps are increased at the boundary between the adjacent cells 55, which causes the super absorbent polymer particles 53 to move easily. Therefore, when the weak bonded portions 54b of dotted line shape are formed by combining wide or narrow line width 54W of the bonded portions 54 and wide or narrow point interval 54D, the weak bonded portions 54b are likely to be peeled off in spite of narrow gaps.

The size of the bonded portion 54 for bonding the upper sheet 51 and the lower sheet 52 can be appropriately determined, and for example, the line width (dimension in the direction orthogonal to the direction surrounding the cell 55) 54W can be about 0.6 to 8.0 mm. In addition, in the case of forming the bonded portions 54 in a dotted line pattern (intermittently in the direction surrounding the cells 55), it is preferable that the length 54L of the bonded portion 54 in the direction surrounding the cell 55 is about 0.6 to 8.0 mm, and the point interval 54D is about 0.8 to 10.0 mm. In particular, in the case of the strong bonded portion 54a, it is preferable that the line width 54W is about 1.0 to 4.0 mm, the length 54L of the bonded portion 54 is about 1.5 to 4.0 mm, and the point interval 54D is about 0.8 to 2.5 mm. Further, in the case of the weak bonded portion 54b, it is preferable that the line width 54W is about 0.6 to 3.5 mm, the length 54L of the bonded portion 54 is about 0.6 to 2.5 mm, and the point interval 54D is about 1.0 to 4.0 mm.

To allow the weak bonded portions 54b to be peeled off, it is possible to determine the type and amount of the super absorbent polymer particles 53 disposed in each cell 55 such that the volume of the super absorbent polymer particles 53 in the cell 55 upon the saturation absorption, becomes sufficiently larger than the volume of the cell 55 adjacent to the weak bonded portions 54b. In addition, to basically prevent the strong bonded portions 54a from being peeled off, it is possible to determine the type and amount of the super absorbent polymer particles 53 disposed in each cell 55 such that the volume of the super absorbent polymer particles 53 included in the unitable cells 55 upon the saturation absorption is smaller than the united volume of the unitable cells 55 due to peeling of the weak bonded portions 54b.

The width of the bonded portion 54 in a case where the bonded portions 54 are formed in a continuous line, and the line width 54W in a case where the bonded portions 54 are formed in a dotted line pattern, are constant in the direction surrounding the cell 55, or also can be changed. In addition, in a case where the bonded portions 54 are formed in a dotted line pattern, the shape of each bonded portion 54 can be appropriately determined, and all of the bonded portions have the same shape, or the bonded portions may have different shapes depending on the site. In particular, when each cell 55 has a polygonal shape, it is preferable to provide the bonded portion 54 at least at one of the intermediate position of each side and each vertex position. Further, in the case of the strong bonded portion 54a, it is preferable to provide it at each vertex position, but in the case of the weak bonded portion 54b, it is preferable not to provide the weak bonded portion 54b at each vertex position, because the weak bonded portion 54b can be peeled off easily and the cells 55 are smoothly coalesced. In a case where the bonded portion 54 is provided at each vertex position, it is desirable that the bonded portion 54 have a radial (star) shape protruding in the direction of each side.

As illustrated in Fig. 8, Fig. 11 to Fig. 15, Fig. 17, and Fig. 18, it is preferable that a longitudinal-strong-bond line 58, in which the strong bonded portions 54a are continuously aligned in the front-back direction LD, and a diffusibility improving portion 57 including low-expanding cells 55s adjacent to both sides thereof are provided in a middle area of the absorber 50 in the width direction WD. The low-expanding cells 55s of the diffusibility improving portion 57 have a smaller amount of contained super absorbent polymer particles 53 per unit area than that of the cells 55 adjacent to both the sides of the diffusibility improving portion 57, and the bonded portions 54 between the low-expanding cells 55s and the cells 55 adjacent to both the sides of the diffusibility improving portion 57 correspond to the weak bonded portions 54b. In this case, as illustrated in Fig. 10, at the beginning of absorption of the excretion liquid, due to a difference in the amount of swelling between the diffusibility improving portion 57 and the surrounding part thereof, a wide groove having the diffusibility improving portion 57 as a bottom is formed, and the groove promotes liquid diffusion. This state continues until the weak bonded portions 54b between the low-expanding cells 55s of the diffusibility improving portion 57 and the cells 55 on both the sides thereof come off due to the swelling forces of the super absorbent polymer particles 53 in the cells 55 around the diffusibility improving portion 57. After the weak bonded portions 54b come off, the strong bonded portions 54a do not come off. Thus, even though the width of the groove decreases, grooves having the strong bonded portions 54a as bottoms remain, and diffusibility is maintained. In other words, in the initial stage of absorption in which diffusion of the large amount of excretion liquid is important, the width of the groove is wide. Thereafter, even though the low-expanding cells 55s of the diffusibility improving portion 57 are united with the surrounding cells 55 so that a problem such as gel blocking does not occur, grooves are left by the strong bonded portions 54a, and the diffusibility improvement effect is maintained.

The amount of the super absorbent polymer particles 53 contained in the low-expanding cells 55s is preferably 1/3 or less of the adjacent cells 55 in terms of weight ratio, and it is particularly preferable that the super absorbent polymer particles 53 are not contained at all in the low-expanding cells 55s.

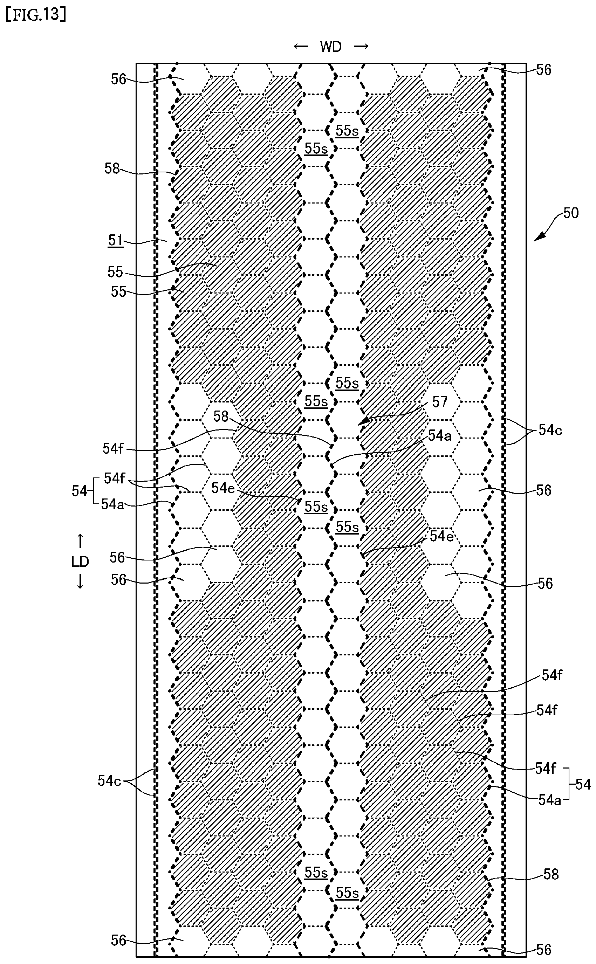

In particular, as illustrated in Fig. 13, when the weak bonded portions 54e between the low-expanding cells 55s of the diffusibility improving portion 57 and the cells 55 adjacent to both the sides of the diffusibility improving portion 57 have higher bonding strength than that of other weak bonded portions 54f and lower bonding strength than that of the strong bonded portions 54a, the weak bonded portions 54e between the low-expanding cells 55s of the diffusibility improving portion 57 and the cells 55 adjacent to both the sides of the diffusibility improving portion 57 are less likely to come off, and the groove formed by the diffusibility improving portion 57 is maintained in a wide state for a longer time, which is preferable. Note that Fig. 8, Fig. 11 to Fig. 15, Fig. 17, and Fig. 18, the strong bonded portions 54a and the weak bonded portions 54e having the relatively high bonding strength are represented by thick dotted lines, the other weak bonded portions 54b and 54f are represented by thin dotted lines, and the cells 55 containing the super absorbent polymer particles 53 (that is, the cells 55 excluding the low-expanding cells 55s and empty cells 56 described later) are hatched.

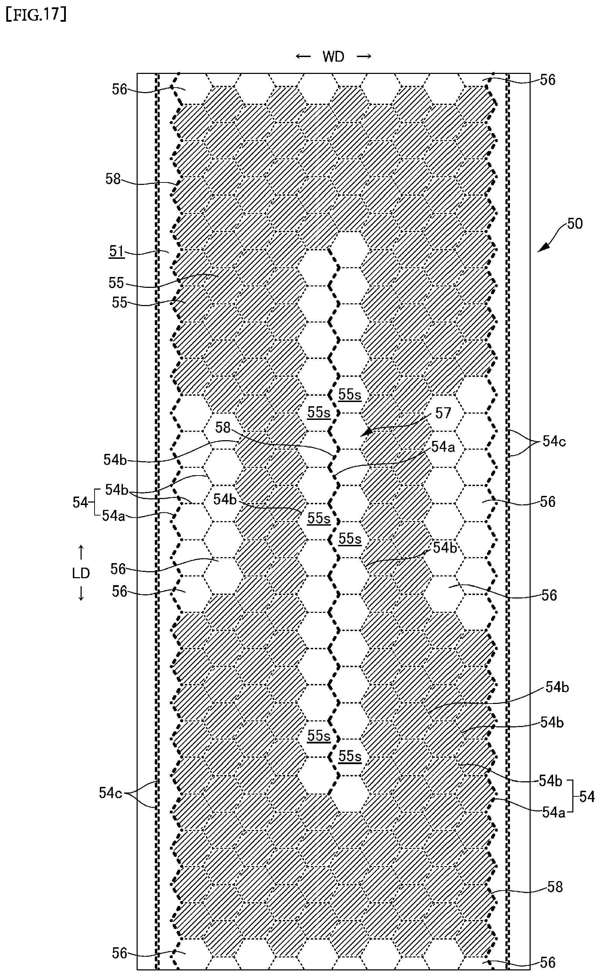

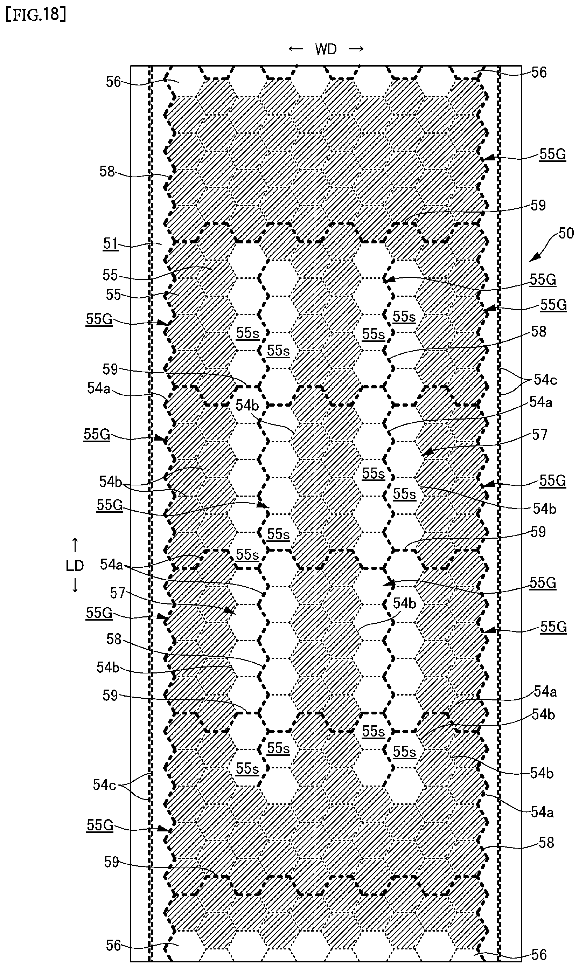

The diffusibility improving portion 57 may be provided over the maximum length of the absorber 50 as illustrated in Fig. 8, and Fig. 11 to Fig. 15, or provided only in an intermediate portion in the front-back direction LD (particularly, a range including the crotch portion and extending over both the front and back sides thereof) as illustrated in Fig. 17 and Fig. 18. In addition, besides providing the diffusibility improving portion 57 at one position at a center in the width direction WD as illustrated in Fig. 8, Fig. 11 to Fig. 15, and Fig. 17, the diffusibility improving portion 57 may be provided at a plurality of positions with intervals in the width direction WD as illustrated in Fig. 18.

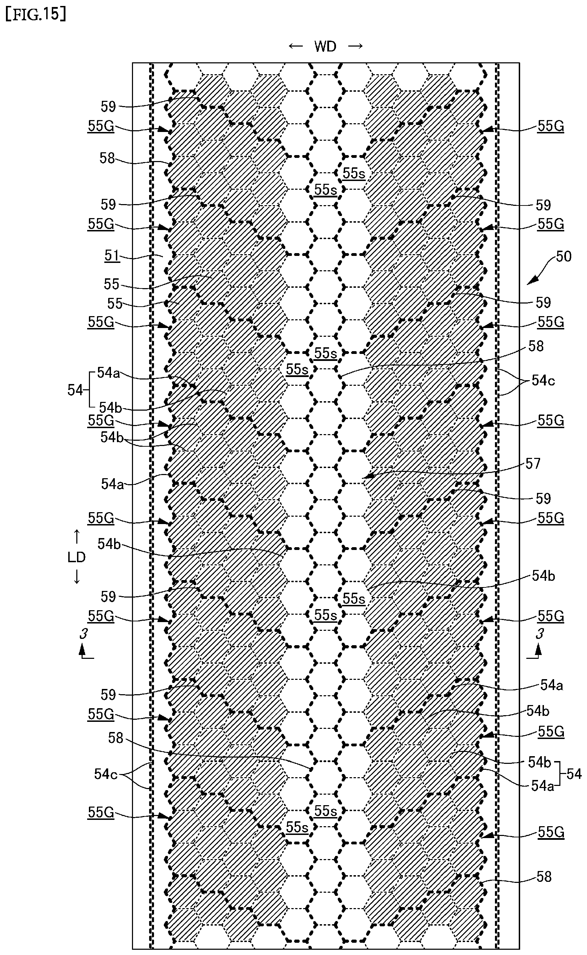

To increase the width of the diffusibility improving portion 57, as illustrated in Fig. 15, a pair of longitudinal-strong-bond lines 58 may be provided along both side edges of the cells 55 arranged in the front-back direction LD, and the cells 55 between the longitudinal-strong-bond lines 58 and adjacent to both sides thereof in the width direction WD may be set to low-expanding cells 55s. By increasing the number of longitudinal-strong-bond lines 58 and the number of low-expanding cells 55s, the width of the diffusibility improving portion 57 can be further increased.

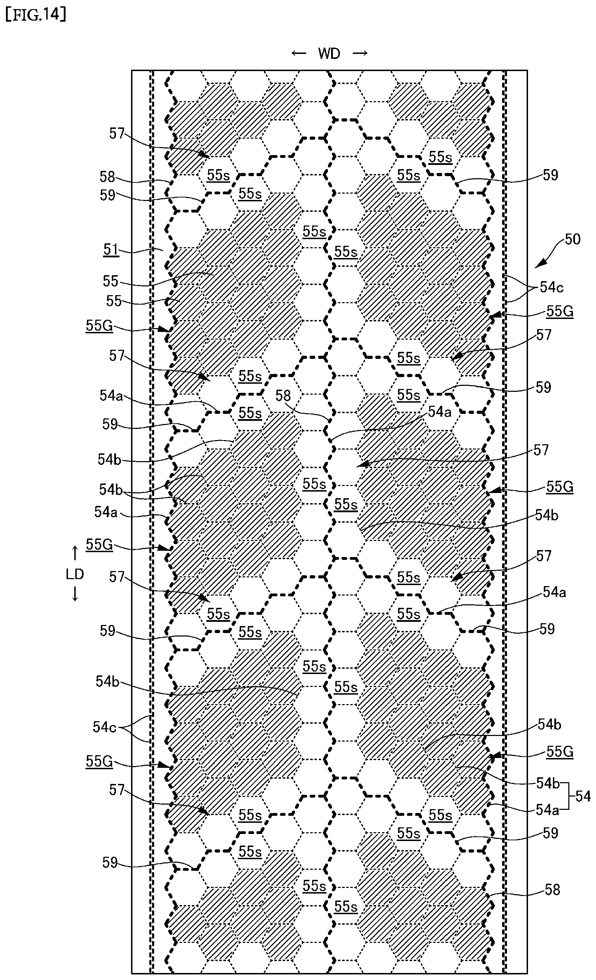

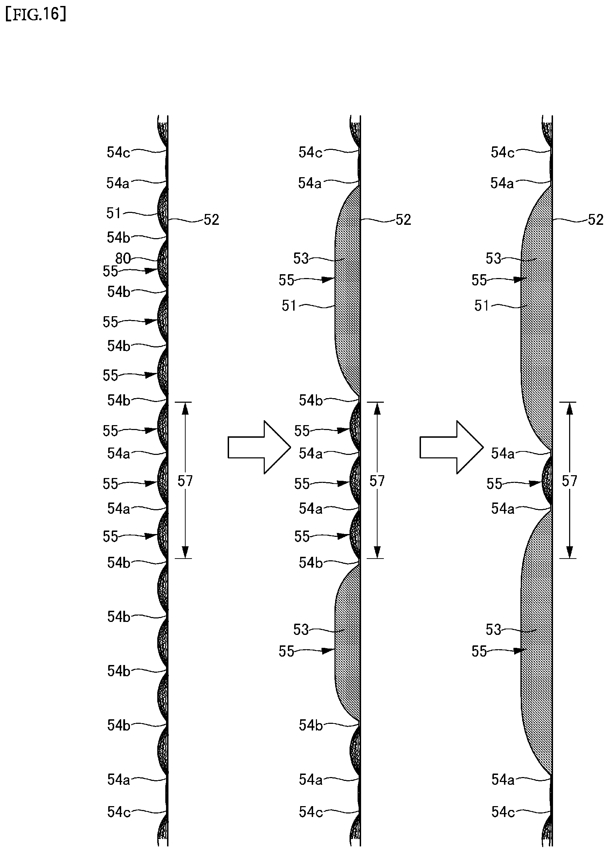

When the cells 55 can be combined with each other over the entire absorber 50 in the front-back direction LD, the gelled material of the super absorbent polymer particles 53 swelling during absorption can largely move in the front-back direction LD in the united cells 55, and there is concern that the gelled material may gather in a low place such as the crotch portion to degrade the wearing feeling. Therefore, as illustrated in Fig. 8, Fig. 11, Fig. 14 to Fig. 16, and Fig. 18, it is a preferable mode that a plurality of lateral-strong-bond lines 59, in each of which the strong bonded portions 54a are continuously or intermittently aligned in the width direction WD or the oblique direction, are provided at intervals in the front-back direction LD. In this way, it is possible to prevent the gelled material of the super absorbent polymer particles 53 from moving in the front-back direction LD by the strong bonded portions 54a that do not basically peel off at the time of absorption, thereby preventing the shape of the absorber 50 from being collapsed. Naturally, as illustrated in Fig. 12, Fig. 13, and Fig. 17, it is possible to adopt a mode not having such lateral-strong-bond lines 59. As illustrated in Fig. 14, the cells 55 adjacent to both front and back sides of the lateral-strong-bond lines 59 can be set to low-expanding cells 55s to provide the diffusibility improving portion 57 in the lateral direction.

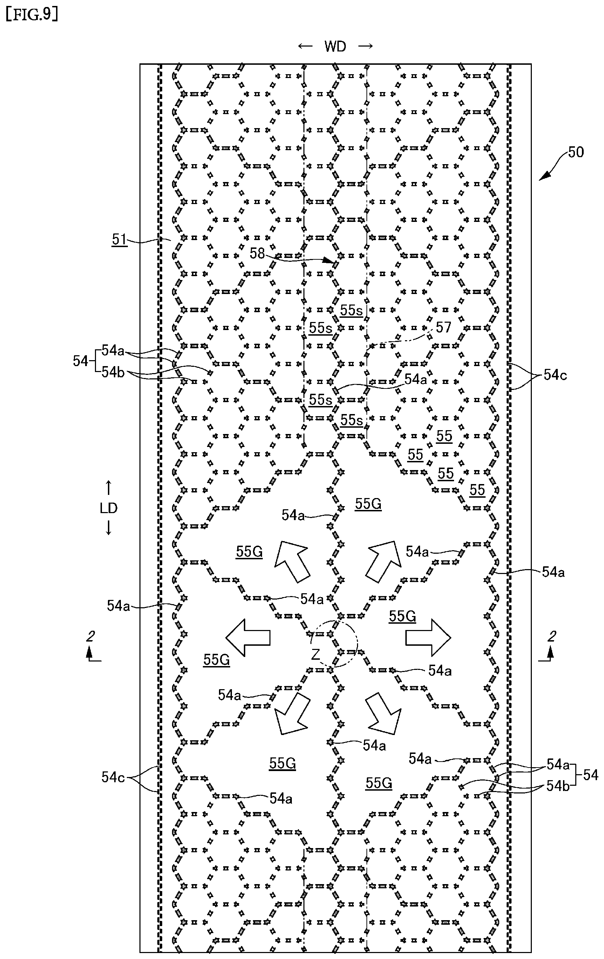

In particular, as in the modes illustrated in Fig. 8, Fig. 11, Fig. 14, Fig. 15, and Fig. 18, when the longitudinal-strong-bond line 58, in which the strong bonded portions 54a continue in the front-back direction LD over the maximum length of the absorber 50, are provided on both sides in the width direction WD along the side edges of the cells 55 located on the outermost side in the width direction WD and provided in the middle thereof in the width direction WD, and the lateral-strong-bond lines 59 are portions continuing in the width direction WD and the oblique direction to extend between the longitudinal-strong-bond lines 58 adjacent in the width direction WD, the cells 55 do not unite with a maximum enlargement section 55G or more surrounded by the strong bonded portions 54a. Thus, the gelled material of the super absorbent polymer particles 53 swelling during absorption does not move to the outside of the maximum enlargement section 55G, and it is possible to effectively prevent shape collapse of the absorber 50 during absorption. In addition, the liquid diffusibility in the longitudinal direction is improved by the longitudinal-strong-bond lines 58 in each of which the strong bonded portions 54a are continuously arranged in the front-back direction LD, and the liquid diffusibility in the lateral direction is improved by the lateral-strong-bond lines 59 in each of which the strong bonded portions 54a are continuously arranged in the width direction WD or oblique direction. For example, in the embodiment illustrated in Fig. 8, assuming that urine is excreted at the position of the reference character Z, the urine is diffused to the periphery around the position as indicated in Fig. 9, and the urine is absorbed in the super absorbent polymer particles 53 at each position. At this time, as illustrated in Figs. 9 and 10, as for each the cell 55, in which the swelling pressure of the super absorbent polymer particles 53 inside is increased, the weak bonded portions 54b surrounding the cell 55 cannot resist against the swelling pressure, and the cell 55 is coalesced with the adjacent cells 55. This coalescence can be continued as long as the weak bonded portions 54b can be peeled off due to the swelling upon the absorption of the super absorbent polymer particles 53 and proceeds to reach the cells 55 having the strong bonded portions 54a positioned in their sides.

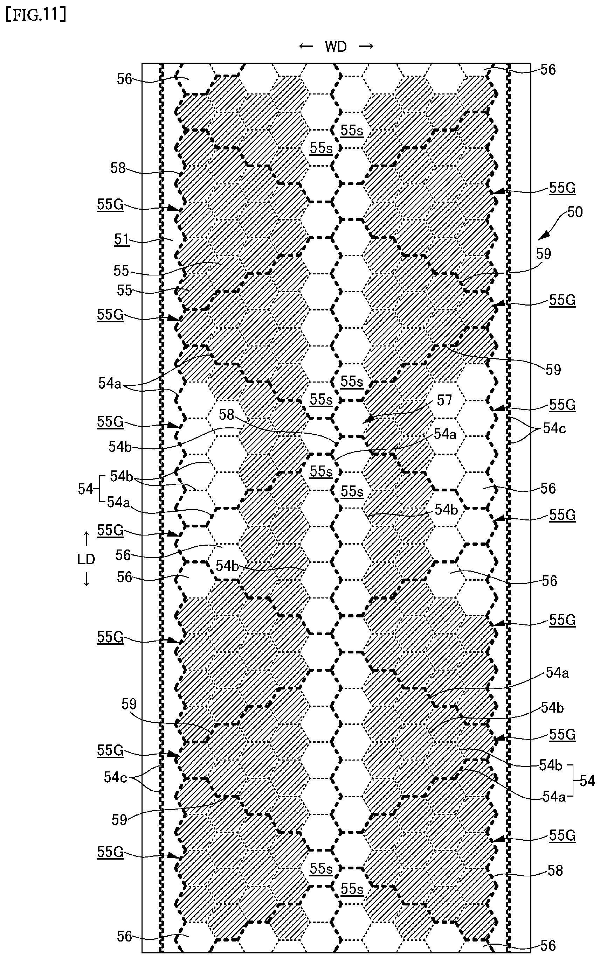

The size, shape, arrangement (that is, arrangement of the strong bonded portion 54a) of the maximum enlargement section 55G can be appropriately determined, but if the maximum enlargement section 55G is too small, it becomes insignificant to provide the strong bonded portions 54a, and even if a large number of the cells 55 are provided, when the maximum enlargement section is formed to be elongated, after the cells 55 are coalesced, the maximum enlargement section comes to have a shape in which it is difficult for the cells to be expanded. For example, when the cell 55 is a regular hexagon as in the illustrated example, it is preferable that, as illustrated in Fig. 11, Fig. 14, Fig. 15, and Fig. 18, in the maximum enlargement section 55G, seven or more cells are arranged in two or more directions, and the peripheral edge of the maximum enlargement section forms a closed shape formed by connecting the sides of the cells 55 along the peripheral edge thereof while two to four consecutive sides are connected per each cell 55. As a result, the cells 55 can be successively coalesced and smoothly enlarged to the maximum enlargement section 55G, and the maximum enlargement section 55G becomes to be expanded easily. When the cells 55 are enlarged to the maximum enlargement section 55G, the increase in the volume of the cell 55 relative to the number of coalesced cells 55 becomes excellent. Note that the term "regular hexagon" includes a deformed hexagon extending and contracting to $\pm 5\%$ in the front-back direction due to an influence of extension and contraction of the upper sheet 51 and the lower sheet 52 during manufacturing.

In the peripheral edge of the maximum enlargement section 55G, a part connecting two consecutive sides per each cell 55 can be a linear part, and a part connecting three consecutive sides per each cell 55 can be linear or a corner portion (turning portion) with an internal angle of 120° and a part connecting four consecutive sides per each cell 55 can be a turning portion with an internal angle of 60° or 180°. Therefore, by combining these linear portions and corner portions, the peripheral edge shapes (arrangements of the strong bonded portions 54a) of the maximum enlargement sections 55G can be formed in substantially equilateral triangular shapes as illustrated in Fig. 11, substantially parallelogram shapes as illustrated in Figs. 14 and 15, or substantially quadrangular shapes as illustrated in Fig. 18.

In the modes illustrated in Figs. 8 to 11, the longitudinal-strong-bond lines 58 are provided at the center and both sides in the width direction WD of the absorber 50, respectively, and the lateral-strong-bond lines 59 are formed in a zigzag shape extending in the front-back direction while repeatedly bending left and right between the longitudinal-strong-bond line 58 in the center portion and each of the longitudinal-strong-bond lines 58 in both the side portions. As a result, the maximum enlargement section 55G having a substantially triangular shape with one of the vertexes positioned in the longitudinal-strong-bond line 58 in the center portion and the maximum enlargement section 55G having a substantially triangular shape with one of the vertexes positioned in each of the longitudinal-strong-bond lines 58 on both the side portions are repeatedly formed alternately in the front-back direction. When the lateral-strong-bond lines 59 are formed in the zigzag shape as described above, it is preferable because the liquid diffusion in the lateral direction can be efficiently facilitated with the small number of the lateral-strong-bond lines 59, and the maximum enlargement section 55G has a substantially triangular shape which is easily expanded, and the increase in the volume of the cells 55 relative to the number of coalesced cells 55 is excellent.

It is possible to use only the longitudinal-strong-bond lines 58 without providing the low-expanding cells 55s. In this case, the strong bonded portions 54a do not come off during absorption of the excretion liquid, and thus the grooves having the strong bonded portions 54a as bottoms are left, thereby improving diffusibility.

On the other hand, as illustrated in Fig. 8, etc., it is also possible to provide the empty cells 56 which contain a smaller amount of the super absorbent polymer particles 53 per unit area than other cells. In Fig. 8, Fig. 11 to Fig. 15, Fig. 17, and Fig. 18, the cells 55 containing the super absorbent polymer particles 53 (that is, cells 55 excluding the low- expanding cells 55s and the empty cells 56 described later) are hatched. Of these cells, an area having a pattern of hatched lines in Fig. 8 is based on assumption of a dispersing region 53A of the super absorbent polymer particles 53 in the manufacturing, and thus there are portions which are not covered by the pattern of the hatched lines in the cells 55 in the peripheral edge. Actually, in a case where the super absorbent polymer particles 53 can move in each cell 55, the positions of the super absorbent polymer particles 53 in the cell 55 are not fixed in a state of the product, and the super absorbent polymer particles 53 can be distributed throughout the cells 55 in the same manner as illustrated in other figures. The amount of the super absorbent polymer particles 53 contained in the empty cell 56 is preferably 1/2 or less of the other cells in terms of weight ratio, and it is particularly preferable that the super absorbent polymer particles 53 are not contained at all in the empty cell. For example, since the front end and the back end of the absorber 50 are formed by cutting into the individual absorbers 50 in the manufacturing, if the super absorbent polymer particles 53 are contained in portions where the cutting is performed, there is concern that the life of a blade of a cutting device may be shortened. Therefore, it is desirable that at least the cells 55 located at the positions through which the front and back ends of the absorber 50 pass be the empty cells 56. Further, in the absorber 50 obtained by mixing the super absorbent polymer particles 53 with a hydrophilic short fiber such as fluff pulp, and accumulating them in a cotton form, generally, the intermediate portion in the front-back direction LD is formed in a narrow shape so as to be along the legs. However, also in the cell 55 absorber 50, by setting, the cells 55 on both the sides in the intermediate portion in the front-back direction LD, as the empty cells 56, the intermediate portion in the front-back direction LD will be less expanded even after absorption, and therefore, the absorber 50 has a shape that fits around the legs even after the absorption.

In the case of manufacturing the cell 55 absorber 50, since it is difficult to accurately distribute a predetermined amount of the super absorbent polymer particles 53 to the individual cells 55, it is preferable that the super absorbent polymer particles 53 are uniformly dispersed throughout the entire region for containing the super absorbent polymer particles 53 (the region excluding the portions to be the empty cells 56) on the upper sheet 51 or the lower sheet 52, and then the bonded portions 54 are formed to bond the upper sheet 51 and the lower sheet 52 as one unit and to confine the super absorbent polymer particles 53 in each cell 55. In this case, particularly with respect to the cells 55 positioned in the peripheral edge of the region for containing the super absorbent polymer particles 53, it is difficult to disperse the super absorbent polymer particles 53 in an accurate shape matching with the peripheral edge of the cells 55. Therefore, as can be seen from the shape of the dispersing region 53A which is defined for dispersing the super absorbent polymer particles 53 and indicated by the pattern of hatched lines in Fig. 8, it is desirable to disperse the super absorbent polymer particles 53 such that the peripheral edge of the dispersing region 53A pass through the middle of the cells 55 located at the peripheral edge of the region containing the super absorbent polymer particles 53. In this case, the amount of the super absorbent polymer particles 53 contained in the cells 55 located at the peripheral edges of the region for containing the super absorbent polymer particles 53 is smaller than that of the cells 55 located on the inside of the cells 55 located at the peripheral edges of the region for containing the super absorbent polymer particles 53. In addition, in the case of having the cells 55 on the outside of the cells 55 located at the peripheral edges of the region for containing the super absorbent polymer particles 53, these outer cells 55 become the empty cells 56 which do not substantially contain the super absorbent polymer particles 53.

In the above example, only the super absorbent polymer particles 53 are contained in the cells 55, but it is also possible to contain the super absorbent polymer particles 53 together with particulate materials other than the super absorbent polymer particles, such as deodorant particles.

The absorber 50 can be wrapped with a wrapping sheet (not illustrated). In this case, one wrapping sheet can be wrapped in a cylindrical shape so as to surround the absorber on the front and back surfaces and both the side surfaces of the absorber 50 and two wrapping sheets can be also wrapped so as to sandwich the absorber from both the front surface side and the back surface side. As the wrapping sheet, tissue paper, particularly crepe paper, a nonwoven fabric, a polyethylene laminated nonwoven fabric, a sheet with small holes, and the like can be used. However, it is desirable that the wrapping sheet be a sheet through which the super absorbent polymer particles do not pass. When a nonwoven fabric is used for the wrapping sheet, a hydrophilic SMS nonwoven fabric (SMS, SSMMS, etc.) is particularly suitable, and polypropylene and polyethylene/polypropylene composite material can be used as a material. The basis weight of the nonwoven fabric used for the wrapping sheet is desirably 5 to 40 g/m$^2$, particularly desirably 10 to 30 g/m$^2$. When the absorber 50 is wrapped with the wrapping sheet(s), pulp fibers can be accumulated on one side of the front surface side and the back surface side of the cell absorber, and the cell absorber together with the pulp fibers can be wrapped with the wrapping sheet(s) collectively.

<Manufacture of absorber>

The above-described absorber 50 can be manufactured by conveying a continuous belt shaped first sheet in a continuous direction, successively forming a large number of recesses at intervals in the CD (Cross Direction) on the first sheet in the conveying process, supplying a particulate material containing the super absorbent polymer particles to the recesses of the first sheet on the downstream side of the recess forming positions, superimposing a belt shaped second sheet continuous in the MD (Machine Direction) on the first sheet on the downstream side of the supply position of the particulate material, bonding a portion between the recesses of the first sheet to the second sheet on the downstream side of a position where the second sheet is superimposed to successively form a continuum of absorbers in which a large number of cells including the particulate material are arranged, cutting the continuum of absorbers into individual absorbers at intervals in the MD, and bonding a belt shaped third sheet continuous in the MD to the second sheet before supplying the particulate material. It is preferable that the first sheet is set to the upper sheet 51 and the second sheet is set to the lower sheet 52 in the above-described absorber 50, but the opposite is also possible. The third sheet corresponds to the middle sheet 80 described above. The third sheet may be bonded to the second sheet before supplying the particulate material or bonded to the first sheet before supplying the particulate material together with or in place of bonding to the second sheet.

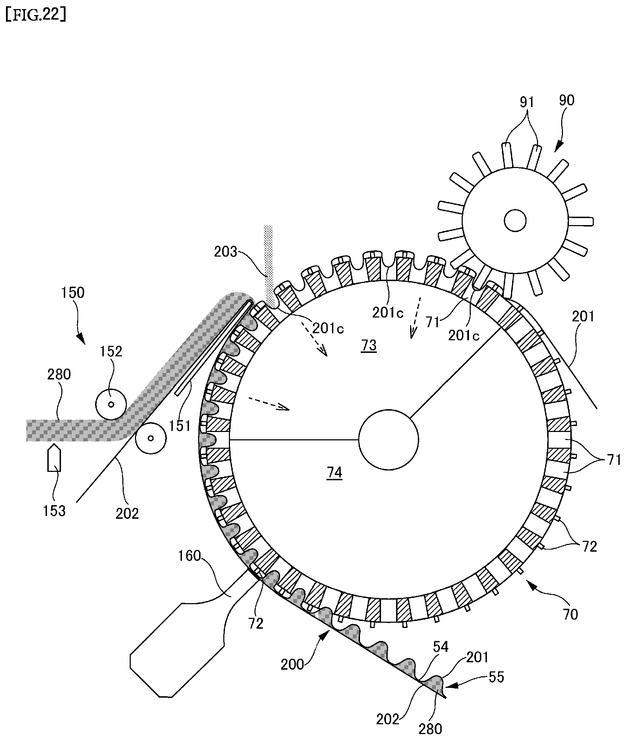

Fig. 22 illustrates a specific example of an apparatus for manufacturing the cell absorber. This manufacturing apparatus is based on an anvil roll 70 driven to rotate about a horizontal rotation axis, and includes first sheet supply means (not illustrated), recess forming means 90, particulate material supply device (not illustrated), and second and third sheet supply means 150 in order from the upstream side in a rotation direction within a rotation direction range of an upper half of the anvil roll 70. In addition, welding means 160 is included on the downstream side of the second and third sheet supply means 150 in the rotation direction.

On an outer peripheral surface, the anvil roll 70 has a large number of concaves 71 arranged at intervals and projections 72 provided at portions between the concaves 71 to surround the respective concaves 71. The concaves 71 on the outer peripheral surface of the anvil roll 70 communicate with an inner space partitioned into an intake section 73 and a non-intake section 74 in the rotation direction, and an intake device such as a suction fan (not illustrated) is connected to the intake section 73 in the inner space of the anvil roll 70 so that the insides of the concaves 71 can be sucked. In addition, an intake port and a fume port are not formed in a portion between the concaves 71 on the outer peripheral surface of the anvil roll 70.

The first sheet supply means supplies a continuous belt shaped first sheet 201 formed of a liquid pervious nonwoven fabric in the rotation direction of the anvil roll 70 so as to be along the outer peripheral surface of the anvil roll 70, and includes various devices such as a guide roll and a drive roll in a path from a raw roll (not illustrated) of the first sheet 201 to the outer peripheral surface of the anvil roll 70.

The recess forming means 90 in the illustrated example faces the anvil roll 70, has push pins 91 entering the respective concaves 71 of the anvil roll 70, and serves as a push roll that forms concaves 201c on the first sheet 201 by passing the continuous belt shaped first sheet 201 in the rotation direction of the anvil roll 70 between the recess forming means 90 and the anvil roll 70 and pushing the first sheet 201 into the concaves 71 using the push pins 91. However, another type of embossing may be applied.

The particulate material supply device can be used without particular limitation as long as the particulate material 203 containing the super absorbent polymer particles 53 is supplied by dropping. Here, the drop supply includes not only a free fall due to the own weight, but also a drop at a speed higher than the free fall. As the particulate material supply device 100, it is possible to use both a device that continuously discharges the particulate material over a whole in the CD at a drop position and a device that can perform intermittent supply for at least a part of a drop outlet in the CD.

The second and third sheet supply means 150 are disposed on the downstream side of the supply position of the particulate material 203 in the rotation direction. The second and third sheet supply means 150 bond a third sheet 280 (refer to the middle sheet 80 described above) having a high porosity to a surface of the continuous belt shaped second sheet 202 formed of the liquid pervious nonwoven fabric on the first sheet 201 side, and then supply the sheet in the rotation direction of the anvil roll 70 along the outer peripheral surface of the anvil roll 70. The second and third sheet supply means 150 include various devices such as a guide roll and a drive roll on a path from a raw roll of the second sheet 202 and a raw roll of the third sheet 280 (not illustrated) to the outer peripheral surface of the anvil roll 70. In the second and third sheet supply means 150 in the illustrated example, first, a hot melt adhesive is applied from a nozzle 153 to a bonding surface of the third sheet 280, and then the third sheet 280 and the second sheet 202 are overlapped and bonded by a bonding roll 152. The hot melt adhesive may be applied to the second sheet 202 instead of the third sheet 280. Subsequently, the second sheet 202 and the third sheet 280 integrated by this bonding are supplied to the anvil roll 70. Here, a guide plate 151 approaching along a tangential direction up to the vicinity of the outer peripheral surface of the anvil roll 70 is disposed, and the second sheet 202 and the third sheet 280 pass above the guide plate 151, are folded at tips thereof, and are then supplied in the rotation direction along the outer peripheral surface of the anvil roll 70. For this reason, a tip of the guide plate 151 is a curved arc surface along a guide direction of the second sheet 202 and the third sheet 280.

The welding means 160 is not particularly limited as long as the welding means 160 welds the first sheet 201, the second sheet 202, and the third sheet 280. In addition to using an ultrasonic horn of an ultrasonic welding device as in the illustrated example, a heating roll (not illustrated) may be used.

In manufacturing, the first sheet 201 is supplied to the anvil roll 70 by the first sheet supply means, and the concaves 201c are successively formed in the first sheet 201 by the recess forming means 90. A degree of a concaves 201c forming processing and wave-form working by the push roll can be determined as appropriate. In normal case, it is desirable that a push depth 91d of the first sheet 201 by the push pins 91 is 2 to 10 mm.

The first sheet 201 on which the concaves 201c are formed rotates and moves to a subsequent supply position of the particulate material supply device 100 while being wound around the anvil roll 70. In this instance, since the concaves 71 are located in the intake section 73 from a stage of forming the concaves 201c, and intake of the concaves 71 is maintained, the concaves 201c are firmly held in the concaves 71 without change from the formation. This intake is maintained at least up to a subsequent supply position of the second sheet 202, preferably up to a welding position. The particulate material 203 is dropped and supplied from the particulate material supply device 100 to the concaves 201c of the first sheet 201. The particulate material 203 can be supplied continuously or intermittently at least in a part in the CD.

The third sheet 280 and the second sheet 202 are immediately wound around the outside of the first sheet 201, on which the particulate material 203 is supplied to the concaves 201c, by the second and third sheet supply means 150, at least a CD range of the first sheet 201 having the concaves 201c is covered with the second sheet 202, and the third sheet 280 is interposed between the first sheet 201 and the second sheet 202. While these first sheet 201, second sheet 202, and third sheet 280 are wound around the anvil roll 70, the portions between the concaves 201c of the first sheet 201, the second sheet 202, and the third sheet 280 located therebetween are immediately welded and bonded at sites of the dot-shaped projections 72 of the anvil roll 70 by the welding means 160, so that a continuum 200 of the absorbers 50 in which a large number of cells 55 including the particulate material 203 are arranged is successively formed. The continuum 200 of the absorbers 50 is sent out from the anvil roll 70, and then cut into individual absorbers 50 at intervals in the MD by a cutter device (not illustrated).

<Explanation of Terms Used Herein>

In a case where the following terms are used in the specification, those have the following meanings unless otherwise specified in the specification.

"MD" and "CD" mean the flow direction (MD) in a manufacturing facility and the lateral direction (CD) orthogonal to the flow direction, and either one is the front-back direction of a product, and the other is the width direction of the product. The MD of a nonwoven fabric is the direction of fiber orientation of the nonwoven fabric. "Fiber orientation" is a direction along which a fiber of a nonwoven fabric runs and determined by, for example, a measurement method in accordance with the fiber orientation test method based on the zero span tensile strength of TAPPI T481 and a simple measurement method for determining the direction of fiber orientation from the ratio of the tensile strength in the front-back direction to the width direction.

"Spread state" means a flatly spread state without contraction or slack.

"Stretch rate" means the value when the natural length is taken as 100%.

"Artificial urine" is prepared by mixing urea: 2 wt%, sodium chloride: 0.8 wt%, calcium chloride dihydrate: 0.03 wt%, magnesium sulfate heptahydrate: 0.08 wt%, and ion exchanged water: 97.09 wt%, and those are used at a temperature of 37°C unless otherwise specified.

"Gel strength" is measured as follows: 1.0 g of super absorbent polymer is added to 49.0 g of artificial urine and the mixture is stirred with a stirrer. The resulting gel is left for three hours in a thermohygrostat bath at 40°C, 60%RH and then cooled to room temperature. The gel strength of the gel is measured with a curd meter (Curdmeter-MAX ME-500, manufactured by I.techno Engineering).

"Basis weight" is measured as follows. After the sample or test piece is preliminary dried, it is allowed to stand in a test room or apparatus under normal conditions (the test location is at a temperature: $23 \pm 1$°C, relative humidity: $50 \pm 2$%) until the constant mass. The preliminary drying is to make the sample or test piece be constant mass in an environment of a temperature of 100°C. The fibers of an official moisture regain of 0.0% do not need preliminary drying. From a test piece having a constant weight, a sample having a size of 100 mm $\times$ 100 mm is cut out using a template for sampling (100 mm $\times$ 100 mm). The sample is weighed and the weight is multiplied by 100 into the weight per one square meter. The resulting value is defined as the basis weight.

"Thickness" is automatically measured under the conditions of a load of 0.098 N/cm$^2$ in a pressurized area of 2 cm$^2$ using an automatic thickness measuring device (KES-G5 handy compression tester).

The "porosity" is measured by the following method. That is, a portion other than the bonded portions of the middle sheet is cut into a rectangle and used as a sample. A length, a width, a thickness, and a weight of the sample are measured. Using the raw material density of the nonwoven fabric, a virtual weight is calculated when the porosity is 0% with the same volume as that of the sample. The porosity is determined by substituting the sample weight and the virtual weight into the following equation.

Porosity = [(virtual weight - sample weight)/virtual weight] $\times$ 100

"Water absorption capacity" is measured according to JIS K7223-1996 "Testing method for water absorption capacity of super absorbent polymers".

"Water absorption rate" is the "time that elapses before the end point" measured in accordance with JIS K7224-1996 "Testing method for water absorption rate of super absorbent polymers" has been carried out using 2 g of superabsorbent polymer and 50 g of physiological saline solution.

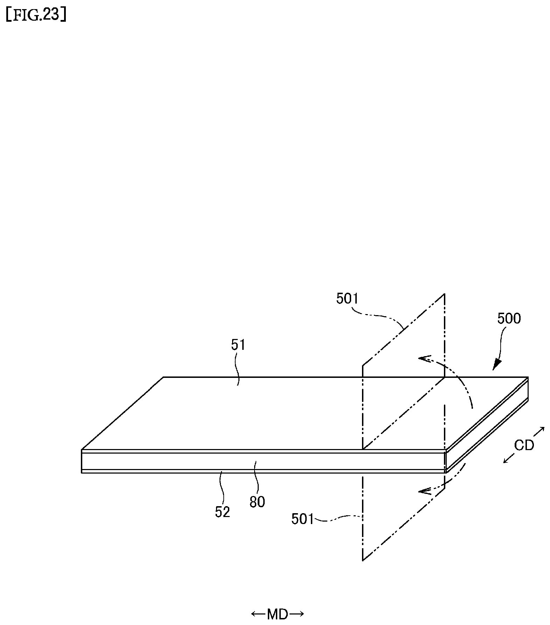

"Peel strength" is measured as follows: That is, as illustrated in Fig. 23, a test piece 500 of 350 mm in the MD $\times$ 40

**EP 3 733 143 B1**

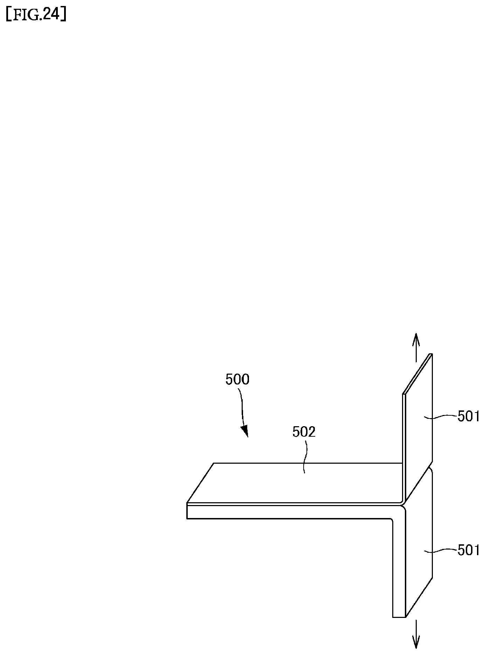

mm in the CD is cut from the absorber 50. Then, a target sheet (the above-described upper sheet 51 and the middle sheet 80, or the middle sheet 80 and the lower sheet 52) in a portion of 100 mm from one end in the MD to a multi-end side of the test piece 500 is peeled off to form a tong hold 501, each tong hold 501 is gripped by upper and lower grips of a tensile testing machine, and a remaining measurement portion 502 is peeled off as illustrated in Fig. 24 under the conditions of gripper spacing of 40 mm and a tensile speed of 300 mm/min, thereby measuring a tensile force (cN/25 mm) required for the peeling. A peeling state of a measurement portion is observed. In the case of interfacial failure (interfacial peeling) and cohesive failure, from a measurement curve having a vertical axis as a tensile force, first five vertices and first five bottom points are selected from a wavy portion after a start of peeling (after the curve rises completely), and an average value of the tensile forces at the respective point is taken as a measured value. In addition, in the case of material destruction (substrate destruction), a maximum value of the tensile force is used as the measured value. The above measurement is performed three times, and the average value is defined as the peel strength. Note that in a case where a size of a test target site is small and a size of the test piece in the CD is less than 40 mm, the peel strength obtained by performing the test in the same manner is converted to about 40 mm.

When environmental conditions in tests and measurements are not described, the tests and measurements shall be carried out in a test room or apparatus under normal conditions (the test location is at a temperature: 23 ± 1°C, relative humidity: 50 ± 2%).

The dimension of each part means the dimension in the spread state, not the natural length state, unless otherwise stated.

Reference Signs List

| | |
|---|---|
| 11 | LIQUID IMPERVIOUS SHEET |
| 12 | OUTER SHEET |
| 12T | TARGET SHEET |
| 13 | FASTENING TAPE |
| 13A | ENGAGEMENT PORTION |
| 13B | TAPE MAIN UNIT SECTION |
| 13C | TAPE ATTACHING PORTION |
| 30 | TOP SHEET |
| 40 | INTERMEDIATE SHEET |
| 60 | THREE-DIMENSIONAL GATHER |
| 62 | GATHER SHEET |
| 50 | ABSORBER |
| 51 | UPPER SHEET |
| 50c | CONCAVE |
| 50d | DEPTH |
| 52 | LOWER SHEET |
| 53 | SUPER ABSORBENT POLYMER PARTICLES |
| 54 | BONDED PORTION |
| 54a | STRONG BONDED PORTION |
| 54b | WEAK BONDED PORTION |
| 54c | EDGE BONDED PORTION |
| 55 | CELL |
| 55s | LOW-SWELLING CELL |
| 55G | MAXIMUM ENLARGEMENT SECTION |
| 56 | EMPTY CELL |
| 57 | DIFFUSIBILITY IMPROVING PORTION |
| 58 | LONGITUDINAL-STRONG-BOND LINE |
| 59 | LATERAL-STRONG-BOND LINE |
| 80 | MIDDLE SHEET |
| LD | FRONT-BACK DIRECTION |
| WD | WIDTH DIRECTION |

**Claims**

**1.** An absorbent article comprising:

19

an absorber (50) including an upper sheet (51), a lower sheet (52) disposed on a back surface side of the upper sheet, a plurality of cells (55) each surrounded by bonded portions (54) of the upper sheet (51) and the lower sheet (52), the upper sheet (51) and the lower sheet (52) not bonded in each cell (55), a particulate material including super absorbent polymer particles (53) contained in each cell (55), and a concave (50c) depressed to outside of each cell (55) in a spread state being formed by embossing the upper sheet (51) at each cell (55),

wherein a middle sheet (80) made of a nonwoven fabric is interposed between the upper sheet (51) and the lower sheet (52),

wherein the middle sheet (80) is compressed in a thickness direction at the bonded portions (54) and is expanded to inside of the concave (50c) within the cell (55), and

(i) wherein a surface of the middle sheet (80) facing the concave (50c) formed in the upper sheet (51) is in contact with an inner surface of the concave (50c) formed in the upper sheet (51), or is spaced apart from the inner surface of the concave (50c) formed in the upper sheet (51) for a separation distance (80s) of 30% or less of a depth (50d) of the concave (50c) formed in the upper sheet (51), and the super absorbent polymer particles (53) in each cell (55) are present most on an upper surface of the middle sheet (80) and decrease downward therefrom.

2. The absorbent article according to claim 1,

wherein the super absorbent polymer particles (53) are not attached to any of the upper sheet (51), the middle sheet (80), and the lower sheet (52), and
the surface of the middle sheet (80) facing the concave (50c) is in contact with the inner surface of the concave (50c).

3. The absorbent article according to claim 1 or 2, wherein the bonded portions (54) are provided in a dotted line pattern along a direction of surrounding the cell (55).

4. The absorbent article according to any one of claims 1 to 3,

wherein the bonded portions (54) include weak bonded portions (54b) allowed to be peeled off by swelling forces of super absorbent polymer particles (53) in adjacent cells (55), and
a peel strength of one of a facing surface between the upper sheet (51) and the middle sheet (80) and a facing surface between the middle sheet (80) and the lower sheet (52) is lower than a peel strength of the other one in the weak bonded portions (54b) wherein the peel strength is measured as described in the description.

5. The absorbent article according to any one of claims 1 to 4, wherein the middle sheet (80) is formed of a nonwoven fabric having a fineness of 1.6 to 7.0 dtex and a porosity of 80 to 98% wherein the porosity is measured as described in the description.

6. The absorbent article according to any one of claims 1 to 5, wherein a ratio of particles having a particle size of more than 500 $\mu$m is 30% by weight or less, a ratio of particles having a particle size of 500 $\mu$m or less and more than 180 $\mu$m is 60% by weight or more, a ratio of particles having a particle size of more than 106 $\mu$m and 180 $\mu$m or less is 10% by weight or less, and a ratio of particles having a particle size of 106 $\mu$m or less is 10% by weight or less in the super absorbent polymer particles (53).

**Patentansprüche**

1. Absorbierender Artikel, umfassend:

einen Absorber (50), der eine obere Lage (51), eine untere Lage (52), die auf der hinteren Oberflächenseite der oberen Lage angeordnet ist, eine Vielzahl von Zellen (55), die jeweils von gebundenen Abschnitten (54) der oberen Lage (51) und der unteren Lage (52) umgeben sind, wobei die obere Lage (51) und die untere Lage (52) nicht in jeder Zelle (55) gebunden sind, ein teilchenförmiges Material, das superabsorbierende Polymerteilchen (53) einschließt und das in jeder Zelle (55) enthalten ist, und eine Höhlung (50c), die außerhalb jeder Zelle (55) in einem ausgebreiteten Zustand eingedrückt ist und durch Prägen der oberen Lage (51) an jeder Zelle (55) gebildet wird, einschließt,

wobei eine aus einem Vliesstoff bestehende mittlere Lage (80) zwischen die obere Lage (51) und die untere Lage (52) eingelegt ist,
wobei die mittlere Lage (80) in einer Dickenrichtung an den gebundenen Abschnitten (54) zusammengedrückt und in das Innere der Höhlung (50c) innerhalb der Zelle (55) ausgeweitet wird, und

(i) wobei eine der in der oberen Lage (51) gebildeten Höhlung (50c) zugewandte Oberfläche der mittleren Lage (80) mit einer inneren Oberfläche der in der oberen Lage (51) gebildeten Höhlung (50c) in Kontakt steht oder von der inneren Oberfläche der in der oberen Lage (51) gebildeten Höhlung (50c) um einen Trennungsabstand (80s) von 30 % oder weniger einer Tiefe (50d) der in der oberen Lage (51) gebildeten Höhlung (50c) beabstandet ist, und die superabsorbierenden Polymerteilchen (53) in jeder Zelle (55) am meisten auf einer oberen Oberfläche der mittleren Lage (80) vorhanden sind und von dort nach unten hin abnehmen.

2. Absorbierender Artikel nach Anspruch 1,
wobei die superabsorbierenden Polymerteilchen (53) nicht an eines aus der oberen Lage (51), der mittleren Lage (80) und der unteren Lage (52) gebunden sind, und die der Höhlung (50c) zugewandte Oberfläche der mittleren Lage (80) mit der inneren Oberfläche der Höhlung (50c) in Kontakt ist.

3. Absorbierender Artikel nach Anspruch 1 oder 2, wobei die gebundenen Abschnitte (54) in einem gepunkteten Linienmuster entlang einer Richtung, die die Zelle (55) umgibt, vorgesehen sind.

4. Absorbierender Artikel nach einem der Ansprüche 1 bis 3,

wobei die gebundenen Abschnitte (54) schwach gebundene Abschnitte (54b) einschließen, die durch Quellkräfte von superabsorbierenden Polymerteilchen (53) in benachbarten Zellen (55) abgeschält werden können, und eine Schälfestigkeit einer von einer zugewandten Oberfläche zwischen der oberen Lage (51) und der mittleren Lage (80) und einer zugewandten Oberfläche zwischen der mittleren Lage (80) und der unteren Lage (52) in den schwach gebundenen Abschnitten (54b) geringer ist als eine Schälfestigkeit der anderen, wobei die Schälfestigkeit wie in der Beschreibung beschrieben gemessen wird.

5. Absorbierender Artikel nach einem der Ansprüche 1 bis 4, wobei die mittlere Lage (80) aus einem Vliesstoff mit einer Feinheit von 1,6 bis 7,0 dtex und einer Porosität von 80 bis 98 % gebildet ist, wobei die Porosität wie in der Beschreibung beschrieben gemessen wird.

6. Absorbierender Artikel nach einem der Ansprüche 1 bis 5, wobei bei den superabsorbierenden Polymerteilchen (53) ein Anteil von Teilchen mit einer Teilchengröße von mehr als 500 μm 30 Gew.-% oder weniger beträgt, ein Anteil von Teilchen mit einer Teilchengröße von 500 μm oder weniger und mehr als 180 μm 60 Gew.-% beträgt, ein Anteil von Teilchen mit einer Teilchengröße von mehr als 106 μm und 180 μm oder weniger 10 Gew.-% oder weniger beträgt und ein Anteil von Teilchen mit einer Teilchengröße von 106 μm oder weniger 10 Gew.-% oder weniger beträgt.

**Revendications**

1. Article absorbant comprenant :

un absorbeur (50) comportant une feuille supérieure (51), une feuille inférieure (52) disposée sur un côté de surface arrière de la feuille supérieure, une pluralité de cellules (55) chacune entourée de parties collées (54) de la feuille supérieure (51) et de la feuille inférieure (52), la feuille supérieure (51) et la feuille inférieure (52) n'étant pas collées dans chaque cellule (55), un matériau particulaire comportant des particules polymères superabsorbantes (53) contenues dans chaque cellule (55), et une concavité (50c) en creux vers l'extérieur de chaque cellule (55) dans un état étalé étant formée en gaufrant la feuille supérieure (51) au niveau de chaque cellule (55), dans lequel une feuille intermédiaire (80) faite d'un non-tissé est interposée entre la feuille supérieure (51) et la feuille inférieure (52),
dans lequel la feuille intermédiaire (80) est comprimée dans une direction d'épaisseur au niveau des parties collées (54) et est étendue vers l'intérieur de la concavité (50c) dans la cellule (55), et

(i) dans lequel une surface de la feuille intermédiaire (80) faisant face à la concavité (50c) formée dans la feuille supérieure (51) est en contact avec une surface intérieure de la concavité (50c) formée dans la feuille supérieure (51), ou est espacée de la surface intérieure de la concavité (50c) formée dans la feuille supérieure (51) pour une distance de séparation (80s) de 30°% ou moins d'une profondeur (50d) de la concavité (50c) formée dans la feuille supérieure (51), et les particules polymères superabsorbantes (53) dans chaque cellule (55) sont le plus présentes sur une surface supérieure de la feuille intermédiaire (80) et diminuent vers le bas à partir de celle-ci.

2. Article absorbant selon la revendication 1,

dans lequel les particules polymères superabsorbantes (53) ne sont fixées à aucune parmi la feuille supérieure (51), la feuille intermédiaire (80) et la feuille inférieure (52), et
la surface de la feuille intermédiaire (80) faisant face à la concavité (50c) est en contact avec la surface intérieure de la concavité (50c).

3. Article absorbant selon la revendication 1 ou 2,
dans lequel les parties collées (54) sont prévues en un motif de lignes en pointillés le long d'une direction entourant la cellule (55).

4. Article absorbant selon l'une quelconque des revendications 1 à 3,

dans lequel les parties collées (54) comprennent des parties collées faibles (54b) qui peuvent être détachées par des forces de gonflement de particules polymères superabsorbantes (53) dans des cellules (55) adjacentes, et une résistance au détachement d'une parmi une surface de face entre la feuille supérieure (51) et la feuille intermédiaire (80) et une surface de face entre la feuille intermédiaire (80) et la feuille inférieure (52) est inférieure à une résistance au détachement de l'autre dans les parties collées faibles (54b), dans lequel la résistance au détachement est mesurée comme décrit dans la description.

5. Article absorbant selon l'une quelconque des revendications 1 à 4, dans lequel la feuille intermédiaire (80) est constituée d'un non-tissé ayant une finesse de 1,6 à 7,0 dtex et une porosité de 80 à 98°%, dans lequel la porosité est mesurée comme décrit dans la description.

6. Article absorbant selon l'une quelconque des revendications 1 à 5, dans lequel un ratio de particules ayant une taille de particules de plus 500 $\mu$m est de 30 % en poids ou moins, un ratio de particules ayant une taille de particules de 500 $\mu$m ou moins et de plus de 180 $\mu$m est de 60 % en poids ou plus, un ratio de particules ayant une taille de particules de plus de 106 $\mu$m et de 180 $\mu$m ou moins est de 10 % en poids ou moins, et un ratio de particules ayant une taille de particules de 106 $\mu$m ou moins est de 10 % en poids ou moins dans les particules polymères superabsorbantes (53).

[FIG.1]

[FIG.2]

[FIG.3]

[FIG.4]

[FIG.5]

(a)

←LD→

(b)

←LD→

[FIG.6]

[FIG.7]

(a)

(b)

[FIG.8]

[FIG.9]

[FIG.10]

[FIG.11]

[FIG.12]

[FIG.13]

[FIG.14]

[FIG.15]

[FIG.16]

[FIG.17]

[FIG.18]

[FIG.19]

(a)

55　　55　　54

(b)

54　55

55

(c)

55　　54

55

(d)

55　55　　54

(e)

54

55　55

[FIG.20]

(a)

(b)

(c)

[FIG.21]

(a)

(b)

(c)

[FIG.22]

[FIG.23]

[FIG.24]

**EP 3 733 143 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014170859 A1
- US 2013079741 A1
- JP 6315501 A
- JP 9504207 A
- JP 2014500736 A
- JP 2011189067 A
- JP 10137291 A
- JP 2017176507 A
- JP 2010522595 A